(19) Europäisches Patentamt
European Patent Office
Office européen des brevets

(11) EP 2 849 769 B1

(12) **EUROPEAN PATENT SPECIFICATION**

(45) Date of publication and mention of the grant of the patent:
**01.07.2020 Bulletin 2020/27**

(21) Application number: **13723395.3**

(22) Date of filing: **20.03.2013**

(51) Int Cl.:
*A61K 36/48* (2006.01) *A61P 1/16* (2006.01)
*A61P 3/10* (2006.01) *A61K 31/047* (2006.01)

(86) International application number:
**PCT/BE2013/000014**

(87) International publication number:
**WO 2013/166563 (14.11.2013 Gazette 2013/46)**

(54) **METHOD FOR PRODUCING A PLANT EXTRACT FROM DESMODIUM AND ITS EXTRACT**

VERFAHREN ZUR HERSTELLUNG EINES PFLANZENEXTRAKTES AUS DESMODIUM UND DESSEN EXTRAKT

PROCÉDÉ POUR LA PRODUCTION D’UN EXTRAIT DE PLANTE À PARTIR DE DESMODIUM ET EXTRAIT CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.03.2012 BE 201200195**

(43) Date of publication of application:
**25.03.2015 Bulletin 2015/13**

(73) Proprietor: **Francis Maes N.V.**
**8400 Oostende (BE)**

(72) Inventors:
- **MAES, Francis**
  **8400 Oostende (BE)**
- **PIETERS, Luc**
  **2000 Antwerpen (BE)**
- **VLIETINCK, Arnold**
  **2920 Kalmthout (BE)**
- **APERS, Sandra**
  **2870 Puurs (BE)**
- **HERMANS, Nina**
  **2980 Zoersel (BE)**

(74) Representative: **Petsis, Christos**
**4-6, Kyparissias**
**542 49 Thessaloniki (GR)**

(56) References cited:
**EP-A1- 0 309 342 WO-A1-2004/084875**

- **MUANDA FRANÇOIS NSEMI ET AL: "Chemical Composition and, Cellular Evaluation of the Antioxidant Activity of Desmodium adscendens Leaves.", EVIDENCE-BASED COMPLEMENTARY AND ALTERNATIVE MEDICINE : ECAM 2011, vol. 2011, 620862, 2011, pages 1-9, XP002687208, ISSN: 1741-4288**
- **BEVERIDGE R J ET AL: "POLY SACCHARIDES OF TROPICAL PASTURE HERBAGE PART 7 IDENTIFICATION OF A NEW PINITOL GALACTOSIDE FROM SEEDS OF TRIFOLIUM-SUBTERRANEUM SUBTERRANEAN CLOVER AND ANALYSIS OF SEVERAL PASTURE LEGUME SEEDS FOR CYCLO HEXITOLS AND THEIR GALACTOSIDES", AUSTRALIAN JOURNAL OF CHEMISTRY, vol. 30, no. 7, 1977, pages 1583-1590, XP009164693, ISSN: 0004-9425**
- **FORD C W: "IN-VITRO DIGESTIBILITY AND CHEMICAL COMPOSITION OF 3 TROPICAL PASTURE LEGUMES DESMODIUM-INTORTUM CULTIVAR GREENLEAF DESMODIUM-TORTUOSUM AND MACROPTILIUM-ATROPURPUREUM CULTIVAR SIRATRO", AUSTRALIAN JOURNAL OF AGRICULTURAL RESEARCH, vol. 29, no. 5, 1978, pages 963-974, XP009164678, ISSN: 0004-9409**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE CA [Online] CHEMICAL ABSTRACTS SERVICE, COLUMBUS, OHIO, US; 31 December 2007 (2007-12-31), MAO, SHAOCHUN ET AL: "Chemical components of Desmodium microphyllum (Thunb.) DC.", XP002687209, retrieved from STN Database accession no. 150:430895
- DATABASE NAPRALERT [Online] 31 December 1982 (1982-12-31), ADINARAYANA D ET AL: "OCCURRENCE OF A RARE DIHOLOSYLFLAVONE, 2"-O-GLUCOSYLVITEXIN IN", XP002687210, retrieved from STN Database accession no. 92:74782 & ADINARAYANA D ET AL: "OCCURRENCE OF A RARE DIHOLOSYLFLAVONE, 2"-O-GLUCOSYLVITEXIN IN", CURR SCI 51 P. 936-937., 1982,
- NARAYANAN C R ET AL: "PINITOL - A NEW ANTI-DIABETIC COMPOUND FROM THE LEAVES OF BOUGAINVILLEA SPECTABILIS", CURRENT SCIENCE, vol. 56, no. 3, 5 February 1987 (1987-02-05), pages 139-141, XP000577900, INDIAN ACADEMY OF SCIENCES, IN ISSN: 0011-3891

## Description

### Field of the Invention

The present invention relates to a method for producing a plant extract from *Desmodium adscendens.*

### Background of the invention

Since ancient times, plants are used for their medicinal properties. Many present drugs are active components of plants such as paclitaxel (Taxol) of *Taxus brevifolia* and morphine of *Papaver somniferum,* or are derived therefrom. In traditional medicine in Africa as well, many plants are used, including *Desmodium adscendens.* Indeed, in terms of geographical distribution and use, the plant is native to many tropical and subtropical countries of Africa and also South America among others, and it grows on open plains, meadows and along the way, making this plant freely accessible. This makes this plant attractive as a drug for populations where medication is often difficult to purchase due to their cost. So, the plant is used in traditional medicine, i.a. for asthma, pain, fever, epilepsy, hepatitis and muscle spasms. For this, a decoct is used as a hot aqueous extract of the leaves, branches or stems.

There are some commercial preparations of flavonoid containing leaf extracts of this plant in circulation that are marketed as dietary supplements with several properties that promote a good health. Also for the treatment of liver diseases, medicinal plants are being used for centuries. So *Silybum marianum, Picrorrhiza kurroa, Curcuma longa, Glycyrrhiza glabra, Phyllantus amarus* and *Andrographis paniculata* are plants which were suspected to have a hepatoprotective effect. The liver is a very important organ in the human body that ensures many important tasks, making liver disorders have a major impact on the body. Indeed, cirrhosis of the liver is one of the major causes of death in the Western world. Further, jaundice is a disorder which occurs because of an increase in non-conjugated or indirect bilirubin, or conjugated or direct bilirubin, and causes yellow discolouration of the skin and sclerae. Cirrhosis is a result of progressive necrosis with scarring (fibrosis), with nodular regeneration occurring between the scars and damaging the normal structure. This results in congestion, portal hypertension, encephalopathy, ascites, etc. From 60 to 70% of liver cirrhosis cases are caused by alcohol abuse. Other causes are viral hepatitis, biliary disease and primary haemochromatosis. Liver failure is the end stage of massive necrosis of the liver caused by viral hepatitis, drugs and chemicals, chronic liver diseases, and of hepatic dysfunction without necrosis, such as Reye syndrome.

To understand the mechanisms of liver injury and to gain insight into the degree of cellular injury, blood parameters are measured, for example the transaminases alanine aminotransferase (ALT) and aspartate aminotransferase (AST). ALT is only present in the cytoplasm, while AST also occurs in the mitochondria. The transaminases rise rapidly, since they are released even during cell wall injury. AST rises later than ALT and indicates that more serious necrosis is occurring. Membrane enzymes such as gamma-glutamyl transferase ($\gamma$GT) and alkaline phosphatase (AP) are present in the cell membranes of almost all of the body's cells. Gamma-GT is elevated in the case of viral or bacterial hepatitis or in the case of intoxication by drugs or alcohol. Alkaline phosphatase is also determined for the diagnosis and follow-up of liver disorders. These parameters provide a picture of the liver injury.

The plant in question *Desmodium adscendens* notably includes flavonoids such as vitexin (1), rutin (2) and isovitexin, the tetrahydroisochinoline salsoline (3), soyasaponins including soyasaponin I (4), $\beta$-phenylethylamines such as tyramine (5) and hordenine (6) shown below under (1) to (6) resp. and an indol-3-alkylamine.

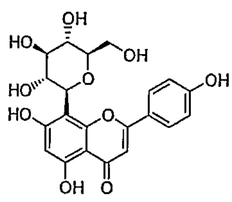

(1)

3

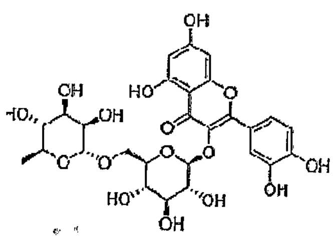

(2)

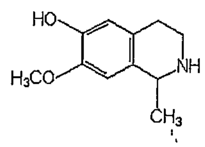

(3)

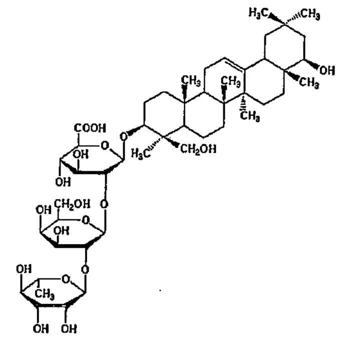

(4)

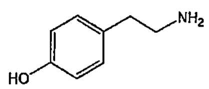

(5)

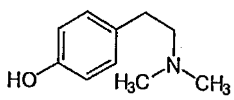

(6)

**Prior Art**

The scientific article MUANDA et al (Evidence-Based Complementary and Alternative Medicine, vol. 2011, 2011, 620862-1 - 620862-9) deals with *Desmodium adscendens* leaves. It indicates a positive effect in case of infections of the liver. An analytical method is yet indicted in which phenolic compounds are identified, and it mentions the presence of certain ingredients but others not, however, despite the central location they will have in this development as will appear further, which therefore puts the importance of this document strongly at issue here, at least based on the crucial constituent referred to here.

Document EP 0309342 A1 of TUBÉRY describes the use of *Desmodium* in the treatment of viral hepatitis of type A or B and toxic hepatitis, but a priori not against all other liver diseases, and drugs for this, in particular for addicted people or cancer treatment. So this document yet also relates to *Desmodium,* particularly *Desmodium adscendens,* with the liver as a target organ for use. A decoct is also mentioned as a form of use, in addition to powder from the dried plant. This document further mentions the principal element that *Desmodium* contains indole alkaloid as an active ingredient. More specifically, this document only relates to an uncharacterized extract of D. adscendens. It provides no further qualitative or quantitative information about the indolalkaloids. In other words, this document provides no validated analytical method for the quantification of the intended crucial constituent here matter. In addition, this document presents *D. adscendens* only as a drug for the treatment of hepatitis.

Until now, liver diseases were very difficult to treat on the one hand, and even almost impossible to avoid, on the other hand. So this is also the problem raised here: how to prevent these liver diseases.

**Aim of the invention**

A principal object of this invention is therefore in the first instance the preparation of an quantified extract from *Desmodium adscendens* that can bring a solutions to the abovementioned problem In this respect, the hepatoprotective properties of extracts of *Desmodium adscendens* are investigated. When looking for a suitable substance for liver diseases, a substance from the group of inositols was tested. These represent a class of compounds which contain hexahydroxycyclohexane derivatives, in particular cyclic sugar alcohols.

Inositols form a part of the ordinary human diet like sugars, without being toxic. Several studies, including in humans, have shown that D-pinitol exerts an effect which is analogous to that of insulin in order to improve the required control of the glycaemy. Said studies indeed suggest that there appears to be a synergy between D-pinitol and insulin at submaximal concentrations, which is not obvious however in glucose transport.

D-pinitol is a cyclic sugar alcohol having a low molecular weight. It is a methyl derivative of D-chiroinositol. Both D-pinitol and D-chiroinositol are structurally related to phosphatidyl inositols, which form a link in the insulin-induced signal transduction. D-pinitol, whose structure is depicted below, occurs in many vegetables, soy products and pine trees -but not exclusively- and it is metabolised in the body to chiroinositol.

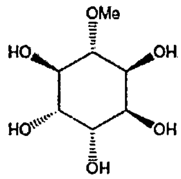

D-pinitol differs from the other inositols because of its specific activity. It is to be understood here that both isomers are meant, thus including the inactive L-pinitol.

A further difference of D-pinitol in regard of the other inositols consists in that it contains a methyl ether group. Given said composition, D-pinitol has a natural influence on the glucose metabolism, and hence on diabetes. *In vitro* pharmacological properties suggest indeed efficacy of D-pinitol including hypoglycemic, antiatherogenic activity and an influence on the immune system. As far as the hypoglycemic activity is concerned, D-pinitol can improve glucose transport and insulin sensitivity. This is owing to the fact that D-pinitol in humans is metabolised partly to D-chiroinositol, which is actually the material that is responsible for the biological activity: D-chiroinositol has an effect on diabetes which is formed by partial metabolism of D-pinitol, the active compound for the positive effects, notably in type-2 diabetes. In other words, insulin sensitivity or resistance is improved. Indeed, it is shown that D-pinitol in man is metabolised to D-chiro-inositol, esp in type-2 diabetes, and it is also extracted unchanged.

In other words, it cannot be deduced at first glance that D-pinitol should come under consideration a *priori* as useful, or even less as an important element for providing a significant contribution to the prevention of the liver diseases discussed here or its treatment.

**Summary of the invention**

Thus according to the invention, a method is proposed for producing a plant extract, quantified for pinitol, wherein a plant from the *Desmodium* family is selected, a fraction is extracted from the *Desmodium* plant parts, and a plant extract is derived from said fraction, which is remarkable in that *Desmodium adscendens* is selected, from which a characterised extract is derived, from which a preparation of this plant extract is quantified for pinitol.

According to this invention, pinitol is proposed as an active main constituent, which plays a crucial role and thus occupies a central position, especially D-pinitol.

MUANDA however mentions no way D-pinitol as constituent of *D. adscendens.*

In contrast, however, document WO 2004/084875 A1 of AMICOGEN does relate to pinitol, or a plant extract containing pinitol for the protection of the liver. However, this document mentions plants as soybean, pine, *Hovenia dulcis, Acanthopanax senticosus* and carob, but again neither *Desmodium,* nor even less *D. adscendens.* Furthermore, this document deals only about uncharacterized extracts. There is no qualitative or quantitative information provided on composition, but only that they contain pinitol or chiroinositol.

The article of BEVERIDGE et al, Aust. J. Chem., 1977, 30, 1583-1590 yet reports the analysis by gas chromatography of D-pinitol in two *Desmodium* types. But this article is about *D. intortum* and *D. uncinatum,* two species that are botanically different from *D. adscendens.*

In an analogous way, the article of FORD et al, Aust. J. Agric. Res. 1978, 29, 963-974 reports the presence of D-pinitol in two *Desmodium* species. But this article is about *D. intortum* and *D. tortuosum,* again two botanical species that are different from *D. adscendens.*

The same is true for Database CA (Chemical Abstracts), which refers to a Chinese journal (Zhongcaoyao 2007, 38 (8), 1157-1159), and reports the presence of pinitol in *D. microphyllum,* which is still further a type which is botanically different from *D. adscendens.*

The same applies again for Database NAPRALERT, which refers to an Indian magazine (Curr. Sci. 1982, 51, 936-937) and reports the presence of (+)-pinitol, i.e. D-pinitol, in *Desmodium triflorum.* However, this article deals with *D. triflorum,* which is again another kind that is botanically different from *D. adscendens.*

In summary, it can be stated that the fact that other *Desmodium* species, different from *D. adscendens,* appear to contain D-pinitol or pinitol, has no predictive value on the presence or absence of this product in *D. adscendens* however. Two different species belonging to the same genus, always have a different chemical profile of secondary metabolites, both qualitatively and quantitatively. There are numerous plant species belonging to other genera and other plant families, which also contain D-pinitol. In the general reference 'Dictionary of Natural Products on DVD', it is explicitly stated "widely distributed in plants" about D-pinitol. This thus implies that there is no consistent or predictable relationship between the genus *Desmodium* and D-pinitol.

One distinguishes the *Desmodium* preparation including pinitol as "whole" or totum, with regard to the above documents, which yet mention the presence of pinitol in other *Desmodium* species, which does not mean although that the occurrence of pinitol in *Desmodium adscendens* can be expected, on the contrary. That seems all too short-sighted.

Indeed, the fact that other *Desmodium* species, which are different from *D. adscendens,* appear to contain pinitol, has no predictive value on the presence or absence of this product pinitol in *D. adscendens*. It is generally known that the medicinal properties of a particular plant species are very often exclusively limited to said only one kind, and these are not present in other species of the same genus and/or family. It is indeed often the case that only a particular plant from a whole plant family is useful for achieving a certain effect, like in this case, *D.A.* from *Desmodium* family is mainly intended against certain diseases as further specified, or also like *Papaver somniferum* versus morphine.

Finally, the publication Short Communication NARAYANAN (Current Science Vol. 56, No. 3, 1987, pp. 139-141) still further relates to pinitol. But it does not address *D. adscendens* but *Bougainvillea spectabilis.*

In addition, MUANDA reports as the most important phenolic component *"quercetin dihydrate",* whereas in Table 1 the terms *"quercetin glucosyl"* and *"quercetin dihydrate"* are used. The presence of vitexin and derivatives thereof is also not reported.

In contrast, however, document WO 2004/084875 relates to pinitol, or a pinitol-containing plant extract for the protection of the liver. This document also reports as plants soybean, pine, *Hovenia dulcis, Acanthopanax senticosus* and carob, but does not discuss *Desmodium,* and thus even less *D. adscendens.* Furthermore, this document describes SOD and glutathione as indicators for the liver-protective effect. So there is no description here of the use of an extract from a D-pinitol containing plant for protecting liver function, which indicates any method for preventing any liver disorder, even less treating same, and certainly not an exotic, i.e., tropical plant with the use of D-pinitol or any extract containing this component.

Furthermore, it relates to hypoglycaemic and anti-diabetic activity, but in no way to a hepato-protective effect, neither to the treatment of a liver disorder.

The presence of pinitol in other *Desmodium* species surely does not detract from the finding that a *D. adscendens* preparation characterised by its content of pinitol has a hepato-protective effect, given that this is not the case for said other *Desmodium* species. In terms of the invention, however, it is true that this allows the preparation of a standardised extract derived from a known plant, *Desmodium adscendens,* quantified for the D-pinitol molecule in its action against hepatitis. The point is that the extraction of D-pinitol from *Desmodium adscendens* discussed here is in no way known, since the related plant *Desmodium adscendens* is used for numerous purposes, even many others than those mentioned here. The scope of the solution which is aimed for, however, lies in the deliberate and targeted selection of a certain indication thereof. There are references yet to numerous products including amino acids, etc., but there is no mention at all of D-pinitol, which is clearly responsible for the activity in the specific plant *D. adscendens* against liver disorders, nor is this suggested, and with the additional advantage that it is present herein in considerable amounts, as a principal constituent thereof, which can furthermore be suitably separated according to the invention.

The invention is defined in the claims.

A first aspect of the invention relates to a method for producing a plant extract, quantified on D-pinitol, wherein a plant *Desmodium adscendens* is selected from the *Desmodium* family, wherein a fraction is extracted from *Desmodium* plant parts, wherein a plant extract is derived from said fraction thereof, characterized in that a characterised extract is derived from *Desmodium adscendens,* from which a preparation of said plant extract is quantified on D-pinitol; wherein said plant extract contains between 0,1 and 10%, preferably from 4 to 5% D-pinitol; particularly wherein a validated analytical method is performed for said quantification of *Desmodium adscendens* on D-pinitol, wherein a specific quantified *Desmodium adscendens* preparation is produced as *"totum",* more particularly wherein a biologically controlled isolation is carried out in said analytical method which is carried out with generating a composition quantified for D-pinitol, wherein the composition product is repeatedly enriched and numerous fractions are isolated, and wherein a decoction is obtained that contains a significant amount of D-pinitol; wherein said extract is separated and the isolated product is identified as the methylated cyclitol 3O-methyl-chiro-inositol, i.e. D-pinitol, in that selected parts of said plant are first dried, and/or in that said plant parts are then ground or fragmented, particularly pulverized into powder form, more particularly wherein said powder is converted as such into a pharmaceutical form; wherein starting from 1 kg of dried plant parts, there is obtained from 60 to 70 g, in particular about 65 g of extract, respectively according to a ratio of 10-20:1, more particularly 14-16:1; in that an aqueous decoction of said plant parts of *Desmodium adscendens* is then prepared warm to a decoct by boiling a certain quantity of dried, optionally powdered, leaves in a certain quantity of water for a certain period of time and in that said aqueous decoction of said plant parts of *Desmodium adscendens* is then cooled, after which the portions obtained are combined and filtered, after which the filtrate obtained is concentrated, in particular under vacuum, and then lyophilized to form a lyophilizate.

A second aspect of the invention relates to a plant extract obtained by the above described method.

A third aspect of the invention relates to a pharmaceutical composition comprising the above plant extract.

A fourth aspect of the invention relates to the above pharmaceutical composition for use as a medicament.

A fifth aspect of the invention relates to the above pharmaceutical composition for use in the treatment of hepatitis, diabetes or metabolic syndrome.

## Brief description of the drawings

Fig. 1 shows a functional working diagram of the method according to the invention in the form of a flow chart; Fig. 2 is a realistic reproduction of a representative sample of leaves and blossoms of *Desmodium adscendens;* Fig. 3 is a schematic representation of insulin signal transduction; Fig. 4 is a graphical representation of the calibration curve obtained after derivatisation with BSTFA; Figs. 5 to 7 are each graphical representations of concentration and area ratios after 3 h and after 6 h, respectively, of derivatisation and after overnight derivatisation; Figs. 8 and 9 are each graphical representations of the concentration and area ratios after 1 h of derivatisation in a heat block; Figs. 10 and 11 are each a graphical representation of the area ratio of D-pinitol/internal standard for 100 mg sample for extraction in an ultrasonic vibration bath; Figs. 12 and 13 are each a graphical representation of the D-pinitol/internal standard area ratio per 100 mg sample for extraction in a heated vibrating bath and by extraction/reflux; Fig. 14 is a graphical representation of the mean area ratios for the various extraction methods; Fig. 15 is a graphical representation of a response function; Fig. 16 is a graphical representation with interferences which gives an overview of residues; Figs. 17 and 18 are each graphical representations of individual measurements and mean values on various days; Figs. 19 and 20 are each graphical representations of values recovered after use of the so-called standard addition method; Figs. 21 and 22 are each a partial chromatogram of analyses respectively without and with internal standard showing the voltage in mV versus minutes; Figs. 23 and 24 are each complete chromatograms of the analysis of the standards showing the voltage in mV versus minutes; Figs. 25 to 28 are each a mass spectrum for various substances and xylitol standards showing the relative influence in per cent versus the most intense signal in terms of specificity; Figs. 29 to 31 show serum AST values 48 h after acetaminophen administration, and serum ALT values 48 and 72 h after acetaminophen administration; Figs. 32 to 38 analogously show additional experimental data, with Figs. 35, 39 showing the further UV spectrum of peaks A-F, Fig. 40 the graphical survival percentage and Fig. 41 an additional chromatographic profile.

## Description

This invention generally relates to a method as defined in the claims, in which the various steps are described schematically and which is used for preparing a special plant extract from *Desmodium adscendens,* the leaves of which serve as the starting product in this production process. *Desmodium adscendens* is a herb that belongs to the family of the *Fabaceae* and the genus

*Desmodium,* a fragment of which is shown in Fig. 2. This is a hardy plant that can grow 0,5-1 m tall and has a round, hairy, vining stem with grooves. The plant is 3-leaved. The supporting leaflets are hairy to hairless at the outer edge and are 0,5-1 mm long and 1,5-3 mm wide. They are winter-hardy. The leaf stalk is hairy and 1-3 cm long. The leaves are elliptical - inverted egg-shaped, blunt and scalloped at the top and wedge-shaped-round at the base. The leaves are primarily hairless on the top and very hairy on the underside.

The manner of blossoming consists of axial and terminal clusters. The leaf stalk is grooved and profusely hairy to fine-haired. The initial bracts are oval-sharp pointed with a pointed top and 3,5-5 mm long and 1,5-2 mm wide. The blossom stalks have the same hair pattern as the leaf stalks and are 0,4-1,7 cm long. The petals are mostly in pairs. The flower crown is larger than the calyx and is oval. The fruit has extended peduncles of 0,5-2 mm long and is 1-5 membered, obliquely elongated with a dimension of 3,5-5,5 mm x 2,5-3 mm. The seed is transversely elliptical and 2,5-5 mm long and 1,5 mm wide.

*Desmodium adscendens* has several pharmacologic properties. In the neuro-pharmacologic area an extract of the plant has a depressive activity on the central nervous system. The ethanolic extract has analgesic and hypothermic activity and inhibits the propagation of tonic-clonic convulsions.

Aqueous and ethanolic extracts of this plant reduce smooth muscle contractions and reduce the release of substances that activate smooth muscle cells in the lungs. Various fractions of the extract are being studied. One sub-fraction inhibits smooth muscle cell contraction induced by antigen via inhibition of phospholipases, which occurs because of activation of calcium-activated potassium receptors. Saponins are present in this fraction. Furthermore, the fraction that contains a tetrahydroisoquinoline analogue inhibits the cytochrome P450 NADPH-dependent mono-oxygenase reaction which produces epoxy- and hydroxyeicosanoids. The fraction increases the COX activity, which results in increased prostaglandin production.

In the experimental phase, first a number of tests were conducted *in vitro,* and then additional ones *in vivo.* The sequence of the test described in the following is shown schematically in the flow chart of Fig. 1.

In a phytochemical study of Desmodium adscendens plant material and extraction thereof from Ghana delivered an aqueous decoction of the leaves, which was prepared by boiling 5x200 g of dried and pulverized leaves in 3 ℓ of distilled water for 1 hour. After cooling, the portions were combined and filtered. The filtrate was concentrated under vacuum and then lyophilized. Starting from 1 kg of dried leaves, approximately 65 g dry decoct was typically achieved.

Then, 20 g of decoct subjected to column chromatography on Sephadex LH20 (120 X 4 cm) with methanol elution. Fractions of 100 ml were collected and analyzed by thin layer chromatography (silica gel Merck, layer thickness

0,25 cm, MeOH/H2 O: 5:1 as mobile phase). Spots were detected under UV light, in particular below a wavelength of 366 nm. After spraying with 1% anisaldehyde / H2SO4 in MeOH, the plate was heated to 120°C for 10 min in order to obtain colored spots. Fractions were combined in 11 sub-fractions according to their chromatographic pattern. Subfractions 5-11 (200mg) showed a spot with a green color, and were pooled. This fraction was subjected to a further column chromatography on Sephadex LH20 (60 x 3 cm) eluted with MeOH, and fractions of 100 ml were collected again and analyzed as described. Sub-fractions 4-5 (120 mg) from this column were combined, and after a new column chromatography under the same conditions, a crystalline product was obtained.

The phase of structural clarification by spectroscopic examination with $^{1}H$, $^{13}C$ NMR and mass spectroscopy and measurement of the specific optical rotation led to identification of the isolated product as the methylated cyclitol 3-O-methyl-chiro-inositol, also known as (+)-pinitol or D-pinitol.

Treatment of 3T3-L1 adipocytes with 0,5 and 1 mM D-pinitol increases the mRNA expression of glucose transporter (GLUT4), insulin receptor substrate (IRS), peroxisome proliferator activated receptor $\gamma$ (PPAR$\gamma$) and CCAAT/enhancer-binding proteins (C/EBP). 1 mM D-pinitol increases expression of adiponectin mRNA, an adipocytokine with anti-inflammatory, anti-diabetic and anti-atherogenic properties, the expression of which is also increased by insulin. The increased expression of a number of factors can be explained by the insulin mimetic properties of D-pinitol.

In L6 rat muscle cells, D-pinitol induces the translocation of GLUT4 to the cell membrane, like insulin, and readies it for the uptake of glucose, see Fig. 3.

With regard to anti-atherogenic activity it was found that D-pinitol moderately decreases the formation of foam cells by reducing the secretion and expression of cytokines such as TNF-$\alpha$, monocyte chemoattractant protein-1, IL-1beta and IL-8 and reducing the expression of macrophage scavenger receptor, CD36 and CD86. The insulin mimetic activity of D-pinitol is probably responsible for this.

Regarding the effect on the immune system, it was found D-pinitol has immunopharmacologic properties and that D-pinitol decreases the expression of MHC-I, MHC-II and co-stimulators such as CD80 and CD86, both in vitro and in vivo, by suppressing MAPKs activation and translocation of NF-kB, and reduces the production of large quantities of IL-12 and pro-inflammatory cytokines in LPS-induced dendritic bone marrow cells. This results in the inhibition of maturation of these cells. Treatment of dendritic cells with D-pinitol prevents these cells from inducing a normal cell-mediated immune response, and when LPS-stimulated dendritic cells are treated with D-pinitol, the proliferation of T-cells and the production of INF-$\gamma$ by CD4+ cells are affected negatively. In neutrophils, D-pinitol inhibits TNF-alpha expression.

D-pinitol inhibits constitutive and induced NF-kB activation in a dose- and time-dependent manner. The inhibition is not cell-specific and takes place through inhibition of IKK activation, IkBa degradation and phosphorylation, nuclear phosphorylation and translocation of p65. D-pinitol also reduces NF-kB-dependent reporter gene expression and suppresses NF-kB-dependent gene products involved in cell proliferation, anti-apoptosis, invasion and angiogenesis. This can explain why analogues of D-pinitol, such as azole nucleoside analogues, have anti-tumour properties. Other derivatives of D-pinitol such as aminocyclitols inhibit glycosidase.

*In vivo* pharmacologic properties were studied in test animals. In the tests conducted *in vivo,* the liver was injured and the preventive and/or curative effects were studied. Until that time it had not been proven that the molecule involved had curative action, although the preventive character thereof was well-proven. In addition to activity in diabetic mice and rats, in streptozotocin-induced diabetic mice - in which D-pinitol has an acute and chronic hypoglycaemic effect - it increases the basal uptake of 2-deoxyglucose in L6 muscle cells by intervening in insulin signal transduction. D-pinitol is not effective in severely insulin-resistant mice. In streptozotocin-induced diabetic rats, D-pinitol lowers blood glucose haemoglobin and increases insulin, while D-pinitol also normalises aspartate transaminase (AST), alanine transaminase (ALT) and alkaline phosphatase values in the liver and has a lipid-lowering effect. The antioxidant effect is manifested in the reduction of lipid peroxidation and hydroperoxidation, an increase in non-enzymatic antioxidants and normalization of the enzymatic antioxidants superoxide dismutase (SOD), glutathione peroxidase (GP), catalase and glutathione-S-transferase (GST).

As far as the hepato-protective effect is concerned, the substance normalises aspartate transaminase (AST) and alanine transaminase (ALT) liver values and TNF-$\alpha$ values after induction of liver injury with galactosamine. In addition, D-pinitol reduces lipid peroxidation and normalises the glutathione, glutathione reductase and glutathione peroxidase values.

It also has an anti-inflammatory effect: in rat studies D-pinitol had anti-inflammatory properties, both against acute (carrageenan-induced oedema of the paw) and subacute (cotton pellet granuloma) inflammation. The substance also has anthelmintic activity.

With regard to the effect on the immune system, in mice with OVA-induced asthma, D-pinitol decreases the number of inflammatory cells in bronchoalveolar lavage fluid and reduces the infiltration of these cells into peribronchiolar and perivascular regions. The inflammation in the lungs is thus combatted. The Th2 cytokines such as IL-4, IL-5 and eotaxins decrease through intake of D-pinitol and the Th1 cytokines such as INF-$\gamma$ increase, as do the INF-$\gamma$ positive CD4 cells. The Th1/Th2 balance is also corrected by D-pinitol through increased expression of the Th1 transcription factor T-bet and a decrease in the transcription factor GATA-3, which is elevated in Th2 pathologies. The gelatinolytic

activity of MMP-9 in lung tissue, which is important in the migration of inflammatory cells from blood to tissue, is decreased. D-pinitol thus reduces the hyperreactivity and inflammation observed in asthma.

**Studies in humans**

In patients with type 2 diabetes, D-pinitol has a favourable effect on fasting glucose values, HbA1c values and insulin. Total cholesterol, LDL/HDL ratio and systolic and diastolic blood pressures decreased after 13 weeks of treatment with 2 x 60 mg D-pinitol per day, while the HDL cholesterol values increased. In patients with uncontrolled type 2 diabetes, 12 weeks of treatment with 20 mg/kg/day of D-pinitol, in addition to the usual therapy, improves the fasting and postprandial glucose values as well as HbA1c values, but does not significantly change the levels of adiponectin, leptin, C-reactive protein (CRP) and free fatty acids. When D-pinitol is taken at a dosage of 20 mg/kg/day for a shorter period of 4 weeks, the substance has no effect on basal and insulin-mediated glucose or lipid metabolism in insulin-resistant patients. In older, non-diabetic patients, D-pinitol intake for 6 weeks has no effect on insulin-mediated glucose metabolism.

The development of an analytical method for D-pinitol in *D. adscendens* decoction is presented in the following with the corresponding validation. Before a standardised preparation can be produced from plant material, the concentration of the active substance in the plant must be known. For this purpose a standard method which is replicable, accurate, not time-consuming and also preferably economical is used. The goal of this master study is the development of an analytical method for determining the content of D-pinitol in a decoction of *D. adscendens.* Various parameters were investigated for this purpose, such as the analytical technique (GC, HPLC, TLC), the column, the detector and the optional purification. Once a possible method is developed, it should also be validated via the ICH standards. Thus the linearity, range, reproducibility, accuracy and specificity will be investigated.

Materials selected included methanol, HPLC grade with analytical quality, pyridine (99+ %), BSTFA 1% TMCS, standards D-pinitol (95%), xylitol (99% minimum) and dulcitol (99+ %), mannitol p.a. and sorbitol, helium, hydrogen gas and air, sodium chloride (p.a.), D(+)-galactosamine hydrochloride (99% minimum) and silymarin.

The decoction of *Desmodium adscendens* was prepared in the following way: 3 litres of distilled water were added to 200 g plant material. The entire product was boiled for 1 h, after which it was cooled and filtered. Volume reduction was accomplished by evaporation using a Rotavapor. As the last step, the decoction was freeze-dried.

In the thin-layer chromatography technique, the stationary phase is on a plastic or glass support. The stationary phase can be silica gel or modified silica, but also aluminium oxide, cellulose or diatomaceous earth.

1-10 $\mu$l of the solution to be analysed is spotted at approximately 1,5 cm from the bottom edge of the plate. Then the plate is placed in a developing tank containing a layer of about 1 cm mobile phase. The mobile phase rises by capillary force and, depending on the type of substances in the solution, the substances elute rapidly or slowly. When the liquid front has almost reached the top edge of the plate, this is removed from the developing tank and the mobile phase evaporated. It is then possible to visualise a spot pattern using UV light or treating with spray reagents and calculate the retention times. A qualitative determination can be performed using a densitometer, which measures the intensity of the spots and converts this into a densitogram.

For the experiments, silica gel $F_{254}$ silica gel TLC-plates, Lichrospher silica gel $F_{254}$ HPTLC-plates and silica gel 60 RP-18 $F_{254}$ plates were used.

Gas chromatography GC is an analytical technique wherein analytes are separated by partitioning between the stationary liquid phase and a mobile gas phase. Because of the high temperature in the injection block, during the injection both the solvent and the analytes evaporate and condense on the cooler column. When the column temperature is increased, the relatively less volatile analytes also enter the gas phase and finally reach the detector. The more non-polar and more volatile the analyte is, the more affinity it has for the gas phase and the shorter is its retention time. More polar or less volatile analytes have more affinity for the more polar stationary phase and reach the detector later. Possible detectors are the flame ionisation detector, electron capture detector, nitrogen-phosphorus detector, katharometer, sulphur detector and mass spectrometer. For these experiments a GC-FID of the type Trace GC Ultra with FID detector was used. To check the specificity, a GC-MS of the type Trace 2000 GC with a Voyager EI MS detector was used.

A flame ionisation detector (FID) and a mass spectrometer (MS) are used in these experiments. The flame ionisation detector is a universal and sensitive detector. The eluate is mixed with $H_2$ and air, and burned. As a result, organic compounds ionise, and the ions increase the current strength relative to a collector electrode at constant voltage. The mass spectrometer works by ionisation of molecules, after which the ions are measured.

Liquid chromatography is an additional analytical technique in which the separation takes place through a difference in distribution between the stationary phase and the liquid mobile phase. If the stationary phase is more polar than the mobile phase, the term "normal phase chromatography" is used, while if the stationary phase is non-polar, *"reversed phase chromatography"* is the term applied. The analytes are eluted with eluent, the composition of which, and thus also the polarity, can be modified to elute analytes more quickly or more slowly. Possible detectors are UV detectors, RI detectors, amperometric detectors, mass spectrometers and evaporative light scattering detectors (ELSD).

An ELSD was used for these experiments, wherein after evaporation of the mobile phase, the light scattering

was measured and converted to an electrical signal.

Possible analytical methods for the analysis of sugars and sugar derivatives, including sugar alcohols such as cyclitols, are known. Several techniques are available for the determination, and a brief overview of these is given below.

For example, for GC techniques, since sugars and sugar derivatives are not volatile, a derivatisation step must also be added here. Both trimethylsilylation and acetylation are known. Both the general flame ionisation detector and the mass spectrometer can be used as detector.

Further, HPLC techniques: since the analytes are not UV-active, they must be derivatised before UV detection is possible. UV-active substances are prepared by, among other things, derivatisation of benzyl chloride or by means of UV-active ion pair reagents. In addition to UV detection, refractive index detection, pulse amperometric detection, mass spectrometry or electron light scattering detection without derivatisation may also be used. Both $C_{18}$-columns and anion exchange columns can be used. If anion exchange columns are used, the sugars are converted to anions with the aid of a base.

Other possible techniques are enzymatic assays, capillary electrophoresis and thin-layer chromatography-densitometry. In thin-layer chromatography as described by Pothier, based on fingerprinting by means thereof, attempts were also made to separate D-pinitol from the other substances in the decoction, since TLC methods are quick and relatively inexpensive.

D-pinitol (standard) and the sample were dissolved in distilled water and 50% methanol-50% water. The mobile phases used were ethyl acetate-formic acid-acetic acid-water (67.5:7.5:7.5:17.5), chloroform-methanol-water (54.5:36.5:9), chloroform-methanol-water (55:36:9), chloroform-methanol-water (46.5:46.5:7) & chloroform-methanol-water (33:53.5:13.5).

D-pinitol was not visible under UV light on the plates with the UV indicator $F_{254s}$, not even at very high concentrations (10 mg/ml), while several spots were seen for the standard when anisaldehyde or thymol was used as the spray reagent.

Therefore the decision was made to develop a method using a different analytical technique. Since sufficient GC methods are described in the literature for analysing D-pinitol, and gas chromatography is a more sensitive technique than that with HPLC derivatisation, the decision was made to develop a GC method.

Based on the determination of D-chiro-inositol in buckwheat using HPLC-ELSD, an attempt was made to develop a liquid chromatographic method in parallel with the gas chromatographic method. Initial experiments indicated that the reproducibility was very low. Therefore this method was not further optimised.

Therefore an immediate change to gas chromatography was made. It is known that various columns can be used for analysis of sugars and sugar derivatives. In the Table below, an overview is given of some of these columns.

Table 1

| | | |
|---|---|---|
| | alkylsilicones | |
| Non polar simplicity | 100% methylpolysiloxane | Inositols and methyl-in *H. rhamnoides* |
| | alkylphenylsilicones | |
| DB-5MS | 5% phenyl polysilphenylene siloxane | Sugars in environmental samples |
| SPB-20 | 20% phenyl 80% methylpolysiloxane | Inositol isomers and arabitol in urine |
| ID.BPx5 | 5% phenyl 95% methylpolysiloxane | Sugars in leaves of *Forsythia* spp. |
| | cyanoalkylsilicones | |
| SPB - 1701 | 14% cyanopropylphenyl 84% methylsiloxane | Polyols in urine |
| DB-225 | 50% cyanopropylphenyl 50% methylsiloxane | Carbohydrates in *D. microcarpum* |
| | packed columns | |
| OV-17 | | Pinitol in soybeans |
| OV-17 (3%) | | Carbohydrates in soybeans |

Columns DB-5 and HP-5 (5% phenyl 95% methylpolysiloxane) and AT™ - 5MS/RTX-5MS (5% phenyl polysilphenylene siloxane), of the Alltech type, which are moderately polar, and the column AT™ - 1/RTX-1 (100% methylpolysiloxane) of the Restek type, which is a column with a non-polar stationary phase, were used. Since a decoction is obtained by boiling plant material in water and thus principally contains polar substances, it was expected that the analytes would have more retention on the 5% phenyl 95% methylpolysiloxane column than the 100% methylpolysiloxane column, and that this would give better separation. Therefore the HP-5 column was chosen over the AT™ -1/RTX-1

column for analysis of the decoction. A column with cyanoalkylsilicones as the stationary phase is the column AT™-264,6% cyanopropylphenyl and 94% methylsiloxane.

An internal standard must also be determined. Since gas chromatography is used here, an internal standard must be added to correct for variations in the injection. An internal standard must behave similarly to the substance being analysed. Therefore it was chosen to use some available substances of structures similar to that of D-pinitol, namely sugar alcohols such as sorbitol, mannitol, dulcitol, xylitol and inositol, which are shown in the following.

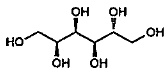
sorbitol

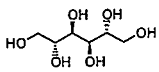
mannitol

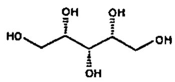
xylitol

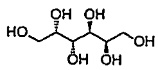
dulcitol

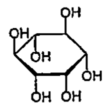
inositol

Monosaccharides such as lactose, however, were not selected since they gave two peaks in the chromatogram as a result of anomerization. This increases the chance of interference with the internal standard; in other words, there is a greater chance that peaks of the sample will overlap with those of the internal standard. In the case of disaccharides, there is always a chance of breakdown, which is difficult to monitor and is undesirable for quantitative analysis. Sugars or sugar derivatives with a high molecular weight compared to D-pinitol elute at a late time and thus extend the duration of the analysis, and therefore this compound was also not selected.

Of the five sugar alcohols selected, dulcitol and inositol are poorly soluble in methanol, but are soluble in water. These substances are not the first choice, since water, even in trace amounts, can cause degradation of the TMS derivatives, which does not help with quantitative analysis. Another advantage is that water takes longer to evaporate to dryness than methanol. Furthermore, very small amounts of water, i.e. trace amounts, are not visible to the naked eye and a drying agent should be used. The influence of this substance on analysis then must also be determined.

Mannitol, sorbitol and xylitol are soluble in methanol, but the retention times of mannitol and sorbitol overlap with those of another unknown in the sample, as is apparent from Table 2 below. Xylitol elutes at a time when no other substance elutes in the chromatogram and only noise is apparent. Structurally also, xylitol is a better choice than mannitol or sorbitol, since xylitol contains the same number of hydroxyl groups as D-pinitol. This is important, since the derivatisation reaction takes place on the hydroxyl groups. Table 2 below contains an overview of the retention times of the possible internal standards.

Table 2

| | Retention time | |
|---|---|---|
| Dulcitol | 24,66 | |
| Inositol | 24,96 | 26,82 |
| Mannitol | 24,46 | |
| Sorbitol | 24,57 | |
| Xylitol | 20,77 | |

As far as the derivatisation is concerned, sugar derivatives can be derivatised to volatile derivatives by acetylation or silylation. Various reagents have been used in the literature, including BSTFA + TMCS in pyridine, HMDS + TFA, HMDS + TMCS in pyridine, STOX + HMDS + TFA, TMSI + pyridine, acetic anhydride + pyridine, and AcO-N-methylimidazole. Among all these possibilities it was decided to perform acetylation with acetic anhydride and pyridine and trimethylsilylation with BSTFA + 1% TMCS (+pyridine) and compare the results.

The acetylation was performed with acetic anhydride and pyridine in a 2:1 ratio. The derivatisation mixture was added to the weighed quantity of solid, D-pinitol, and sorbitol was added as internal standard. In this process it was necessary to search for the correct volume of derivatisation mixture so that D-pinitol would be soluble in it. The mixture was either heated for 30 minutes in the oven at 60°C, or the vial was stored overnight at room temperature. After derivatisation the samples were evaporated to dryness under a nitrogen stream, after which the derivatives were redissolved in ethyl acetate and analysed. An AT™-264 column was used for this purpose, in analogy to the literature where a DB-225 column was used for analysis of acetylation derivatives (Ac20- N-methylimidazole). The temperature of the oven was 200°C and was raised at 5°C/minute to 220°C, which was maintained for 20 minutes.

The peaks showed a very great variation with regard to retention time, and the reproducibility of the peak areas left something to be desired. One explanation might be that the derivatives, from a relative viewpoint, are not volatile enough, since 5 hydroxyl groups had to be derivatised.

When trimethylsilylation was used, D-pinitol and sorbitol were weighed out and dissolved in 2 ml methanol.

Sorbitol was used for the first derivatisation experiments, and then later the internal standard was chosen as described above. 100 µl were evaporated to dryness under a stream of nitrogen, with 100 microlitres of BSTFA + 1% TMSC and 40 µl of pyridine being added. The vial was held for 3 h in an oven at 70°C. Then the derivatisation reagent was evaporated off and the residue was re-dissolved in 300 µl hexane. Each level was weighed once but derivatised in duplicate and injected in triplicate. The reaction of the derivatisation of a substance containing a hydroxyl group with BSTFA is shown below:

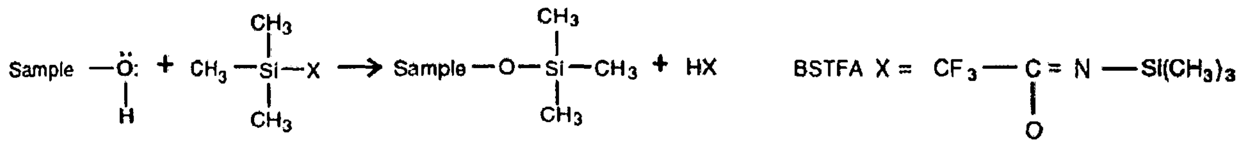

Table 3 below gives an overview of the concentration ratio & the area ratio for D-pinitol/internal standard.

Table 3

| Ratio of concentrations | Ratio of areas |
|---|---|
| 0,29 | 0,25 |
| 0,96 | 0,83 |
| 1,74 | 1,38 |

Fig. 4 shows a calibration curve obtained after derivatisation with BSTFA, which appears linear. At first glance this method appears linear and the retention time also remains the same. It was possible to determine from the results of the initial experiments that the derivatisation method is more successful than acetylation. Therefore this derivatisation method was used further.

Then a further look was taken at derivatisation with xylitol as the selected internal standard. Furthermore it was determined whether the reaction time causes any change in the areas.

A methanolic solution of D-pinitol and xylitol was prepared with batches of respectively (25 mg/50 ml) and (10 mg/50 ml). In 10 ml volumetric flasks, 2 ml internal standard solution was placed and various quantities of D-pinitol were added. Then these were diluted. 500 µl of this solution was evaporated to dryness under a stream of nitrogen. Then 0,1 ml of derivatisation mixture was added to each vial and the vials were placed in an oven at 70°C for 3 hours, 6 hours and overnight. Then the derivatisation reagent was evaporated off, the residue re-dissolved in 300 µl hexane and injected into the GC in duplicate.

Table 4 below gives an overview of the concentration ratio and area ratio after three hours of derivatisation, showing linearity.

tabel 4

| Concentratie D-pinitol I/IS | oppervlakte D-pinitol I/IS reeks1 | oppervlakte D-pinitol I/IS reeks2 |
|---|---|---|
| 0,741 | 0,746 | 0,870 |
| 1,185 | 1,324 | 1,292 |
| 1,481 | 1,318 | 1,302 |
| 1,778 | 1,654 | 1,595 |
| 2,074 | 1,862 | 2,059 |
| 2,370 | 2,084 | 2,198 |
| 2,951 | 2,892 | 3,550 |

tabel 5

| Concentratie D-pinitol I/IS | oppervlakte D-pinitol I/IS reaks1 | oppervlakte D-pinitol) I/IS reeks2 |
|---|---|---|
| 0,741 | 1,374 | 1,303 |
| 1,185 | 1,041 | 1,212 |
| 1,481 | 1,356 | 1,498 |
| 1,778 | 1,676 | 1,723 |
| 2,074 | 1,905 | 2,263 |

(continued)

| Concentratie D-pinitol I/IS | oppervlakte D-pinitol I/IS reaks1 | oppervlakte D-pinitol) I/IS reeks2 |
|---|---|---|
| 2,370 | 2,106 | 2,920 |
| 2,951 | 2,849 | 2,599 |

Fig 5. is a graphical representation of the concentration & area ratios after 3 hours of derivatisation.

Table 5 above gives an overview of the concentration and area ratios after 6 hours of derivatisation, while Fig. 6 is a graphical representation of the concentration and area ratios after 6 hours of derivatisation.

Table 6 below gives an overview of the concentration and area ratios after overnight derivatisation, while Fig. 7 is a graphical representation of the concentration and area ratios after overnight derivatisation.

Table 6

| Concentration of D-pinitol I/IS | Area of D-pinitol I/IS Series 1 | Area of D-pinitol I/IS Series 2 |
|---|---|---|
| 0,741 | - | 0,840 |
| 1,185 | 1,200 | 0,977 |
| 1,481 | 1,353 | 1,432 |
| 1,778 | 2,407 | 1,671 |
| 2,074 | 1,905 | 1,916 |
| 2,370 | 2,215 | 2,232 |
| 2,951 | 2,718 | 2,647 |

Regardless of the derivatisation time there is great intervariability for a point with the same concentration, as was apparent from Fig. 5 to 7 and the 3 tables above. No conclusions could be drawn from this regarding the completeness of the derivatisation reaction. Another problem was that when samples were left in the oven overnight, some of the vials were empty in the morning, and the derivatisation reagent had apparently evaporated. This is also not desirable for a quantitative measurement. For practical purposes it is extremely difficult to allow samples to derivatise for 6 hours.

Then the choice was made to use a heating block to prevent this. Reaction vials, which conducted the heat better, were also used. One hour of derivatisation should be adequate with this method. From a solution of D-pinitol and xylitol in respective quantities of 12,5 mg/100 ml and 10 mg/50 ml, 2 ml internal standards and different quantities of D-pinitol were pipetted into 10 ml volumetric flasks and diluted. 50 μl of this solution was evaporated to dryness under a nitrogen stream. 50 μl of derivatisation mixture was added to each vial and the vials were placed in the heating block for 1 hour at 70°C. Then 100 μl of hexane were added, vortexed and injected into the GC in duplicate. Table 7 below shows an overview of the concentration and area ratios after 1 hour of derivatisation in a heating block, while Fig. 8 is a graphical representation thereof.

| Concentratie D-pinitol I/IS | surface D-pinitol I/IS series | surf D-pinitol I/IS. |
|---|---|---|
| 0,741 | 0,658 | 0,681 |
| 1.185 | 1,039 | 1,040 |
| 1,481 | 1,299 | 1,297 |
| 1,778 | 1,559 | 1,567 |
| 2,074 | 1,840 | 1,832 |
| 2,370 | 2,099 | 2,099 |
| 2,951 | 2,508 | - |

| Concentratie D-pinitol I/IS | surface D-pinitol I/IS ser.1 | surface D-pinitol I/IS ser.2 |
|---|---|---|
| 0,408 | 0,345 | 0,349 |
| 0,653 | 0,551 | 0,551 |
| 0,817 | 0,690 | 0,690 |
| 0,980 | 0,825 | 0,826 |
| 1,144 | 0.963 | 0,966 |
| 1,307 | 1,098 | 1,095 |

(continued)

| Concentratie D-pinitol I/IS | surface D-pinitol I/IS ser.1 | surface D-pinitol I/IS ser.2 |
|---|---|---|
| 1,634 | 1,365 | 1,368 |

In a subsequent experiment, a higher concentration of xylitol was used and no further hexane was added after the derivatisation reaction was complete. In this way it was possible to avoid an extra step in the analytical procedure. Table 8 above shows an overview of the concentration and area ratios after 1 hour of derivatisation in a heating block.

When the derivatisation reaction is performed in reaction vials and in a thermal heating block, there are no longer any outliers among the points on the calibration curve. Thus there is a clear difference with respect to the use of the oven. Comparing the two graphics it is seen that the addition of hexane has a great influence on the method. Nevertheless, it was chosen to skip this step in order not to use solvent unnecessarily and to make the analysis less labour-intensive.

Now the extraction: to work quantitatively, the extraction of the sample must also be complete. In the following experiment the best extraction method was sought.

In a first experiment, extraction was performed with an ultrasonic vibrating bath. For this purpose in each case approximately 100 mg of sample was weighed out. In this case, 2 ml of internal standard xylitol (18 mg/50 ml) were added and diluted to 10 ml with methanol. 50 μl of this was evaporated to dryness, derivatised and injected into the GC. In the table below an overview is given of the area ratio of D-pinitol/xylitol for extraction 2, 3 or 4 times and when a larger volume is used. Table 9 below shows an overview of area ratio of D-pinitol/internal standard per 100 mg sample after various numbers of extractions on an ultrasonic vibrating bath, while Fig. 10 is a graphical representation thereof, in which 1 represents 2x extraction, 2 represents 2x extraction in 20 ml versus 10 ml, 3 represents 3x extraction and 4 represents 4x extraction, respectively.

Table 9

| | 2x extraction | 2x extraction (20ml) | 3x extraction | 4x extraction |
|---|---|---|---|---|
| **1** | 0,592 | 0,623 | 0,640 | 0,689 |
| **2** | 0,588 | 0,631 | 0,659 | 0,683 |
| **mean** | 0,590 | 0,627 | 0,650 | 0,686 |

From the above table and Fig. 10, it can thus be concluded that the extraction is not yet complete after two or even three extraction cycles. To find out whether extraction is complete after four cycles, it would be necessary to perform a fifth cycle. This process was too labour-intensive, and therefore further study was conducted on how the substance can best be extracted. A larger volume also plays a role; when the volume is doubled, the area ratio likewise increases.

In the next experiment the samples were dissolved in 50 ml and 100 ml methanol, together with the same amount of internal standard as before. The samples were vibrated for 30 min or 1 hour on the ultrasonic vibrating bath. Then a certain quantity was evaporated to dryness, derivatised and analysed. In Table 10 below the data are shown, summarising the area ratio of D-pinitol/internal standard per 100 ml sample after various extraction cycles on an ultrasonic vibrating bath.

Table 10

| | 50 ml - 30 min | 100 ml - 30 min | 50 ml - 1 h | 100 ml - 1 h |
|---|---|---|---|---|
| **1** | 0,488 | 0,492 | 0,627 | 0,620 |
| **2** | 0,530 | 0,535 | 0,542 | 0,568 |
| **mean** | 0,509 | 0,514 | 0,585 | 0,594 |

Fig. 11 is a graphical representation of this, in which 1 represents 50 ml-30 min, 2 represents 50 ml-1 h, 3 represents 100 ml-30 min and 4 represents 100 ml-1 h, respectively.

When a volume of 50 ml is chosen, the ratio does not increase further when the volume is increased. However, there is a significant difference between extracting for 30 minutes or 1 hour, as Table 10 and Fig. 11 show. Nevertheless, the extraction in this way is still not complete, since the ratio is still less than that after four extractions in 10 ml methanol.

It is apparent from the preceding experiments that principally the time versus the number of extractions is an important factor. However, when a sample is in the vibrating bath, this also heats up, so that the heat factor should also be pursued. Table 11 below gives an overview of the D-pinitol/internal standard area ratio for a 100 mg sample after

various extraction cycles in a heated ultrasonic vibrating bath.

Table 11

| | 1x extraction | 2x extraction | 3x extraction | 4x extraction |
|---|---|---|---|---|
| **1** | 0,634 | 0,670 | 0,646 | 0,670 |
| **2** | 0,631 | 0,702 | 0,716 | 0,716 |
| **mean** | 0,632 | 0,686 | 0,681 | 0,693 |

Fig. 10 is a graphical representation of this, wherein 1 represents 1x extraction, 2 represents 2x extraction, 3 represents 3x extraction and 4 represents 4x extraction, respectively.

On average, extraction in a heated vibrating bath provides a maximum yield after 2x extraction. After 1, 3 or 4 extraction cycles the ratio does not increase further. The ratio after two cycles of heated ultrasonic vibration is considerably greater than after 1x 1 hour of extraction without heat, as would have been expected based on Table 11 and Fig. 11. To pursue the effect of heat, another technique, refluxing, was also used. The table below gives an overview of the area ratio of D-pinitol/internal standard per 100 mg sample after various extraction cycles on a heated ultrasonic vibrating bath.

Table 12

| | 1x refluxing | 2x refluxing | 3x refluxing | 4x refluxing | 1x 1 h refluxing |
|---|---|---|---|---|---|
| **1** | 0,708 | 0,711 | 0,674 | 0,718 | 0,722 |
| **1b** | 0,717 | - | - | - | - |
| **2** | 0,709 | 0,698 | 0,722 | 0,660 | 0,710 |
| **mean** | 0,711 | 0,705 | 0,698 | 0,689 | 0,716 |

The ratios obtained by refluxing are similar after 1, 2, 3 and 4 cycles of refluxing and also when refluxing is performed once for 1 hour as shown in Table 12 and Fig. 13. To approach the range of these values it would be necessary to perform vibration at least twice in an ultrasonic vibrating bath. The values obtained by refluxing are also just a bit higher. Whether this is coincidence cannot be determined from these two experiments. Nevertheless, it is apparent that refluxing is the most productive and least labour-intensive extraction method, as shown in Fig. 14.

In the development of a final method, initially a look was taken at the internal standard solution, wherein 10,5 mg xylitol was weighed into a volumetric flask on a balance and dissolved in methanol. It was diluted to 100 ml. 25 ml of this solution were pipetted into a 250 ml volumetric flask and diluted to 250 ml. The quantity of internal standard gave a D-pinitol/xylitol ratio in the sample = 1.

Then for sample preparation 100 mg of the decoction was weighed into a round-bottom flask. Then 50 ml of the internal standard solution was added. This mixture was refluxed for 30 min. After cooling this was transferred to a tube and centrifuged for 5 minutes at 3000 g. The supernatant was transferred to a receiving vessel. Then 150 μl of this was pipetted into a reaction vial and evaporated to dryness under a stream of nitrogen. Then 50 μl of derivatisation reagent (BSTFA 1% TMCS - pyridine 2:1) were added and vortexed. The reaction vials were heated for 1 h in the heating block at 70°C. After cooling the vials, the contents were transferred to vials that are compatible with the GC auto-injector.

The following temperature gradient was used for the analysis: the temperature was 65°C for the first two minutes, then the temperature was raised to 300°C at the rate of 6°C/min. Then 300°C was maintained for 15 minutes. A gas with a flow rate of 1,3 ml/min was used.

Now the validation of the method. The validation of analytical methods is performed according to the ICH guidelines. According to these guidelines, the linearity, reproducibility and intermediate precision, accuracy, specificity and range of an assay are to be evaluated.

In determination of the calibration model and range, linearity is defined in that results are obtained which are equivalent to the concentration of the analyte in the sample. The range is the interval between the lowest and the highest concentration of analytes within which it is shown that the analytical method is accurate, precise and linear.

The response function was determined by injecting 5 standards at concentrations between 40 and 200% of the theoretical value in duplicate. To determine whether the calibration model is linear, the calibration curve is inspected visually and a linear regression analysis is performed. Fig. 15 shows that the response curve appears linear and thus is a straight line.

**Table 13**

| t-test on the slope | |
|---|---|
| **Theoretical t-value** | **2,2281392** |
| **Calculated t-value** | **119,3243** |

| 95% CI intersection point (0,0 | |
|---|---|
| **Lowest 95%** | **Highest 95%** |
| **0,000770923** | **0,048859935** |

Table 13 illustrates the application of regression analysis with a t-test on the slope and 95% confidence interval intersection point (0,0). It is apparent from the regression analysis that the correlation coefficient, 0,999298, is high enough, in other words >0,99. It is apparent from Table 13 that there is a significant slope from the right, which is also apparent visually in Fig. 15. When the 95% confidence interval is calculated for the intersection point it is clear that the straight line does not pass through (0,0).

The residuals $y_i$ - <$y_i$> are plotted against $x_i$ or <$y_i$> to determine whether the residuals are randomly distributed, in other words, that homoscedasticity applies. Fig. 16 shows that the residues are uniformly distributed and the model is homoscedastic. The residue with the greatest deviations still has a deviation of less than 5% relative to the expected value.

An ANOVA lack of fit test was performed to determine whether the model is correct. When the average of the two measured values -for each concentration- deviates too much from the calibration curve with respect to the variance between the two measured values, the F-value would be greater than the critical one, and the model is erroneously selected. The calculated F value is 0,7 and therefore smaller than 4,534. All these data show that the calibration model is linear.

With regard to the precision or repeatability, when analyses are performed with a given method on the same sample, it is expected that the method always gives the same results. For this, the precision or repeatability of the method is verified on different levels. The repeatability of the injection was determined by injecting the same sample 6 times. On three different days, 6 samples were analyzed so as to check repeatability within a day and the intermediate precision.

Also at different concentration levels, namely the lowest and the highest concentration in the range, e.g. 50% & 200% of the theoretical value, the precision was analyzed.

In order to analyze the repeatability of the injection, the following parameters were calculated from the measurement results: the average, the standard deviation and the relative standard deviation.

Table 14 below shows the D-pinitol rates for six injections of the same sample with mean, standard deviation and relative standard deviation expressed in %. It follows from this table that the standard deviation and relative standard deviation are very small, i.e. that the injection is repeatable.

Table 14

| Rate D-pinitol (%) | |
|---|---|
| 1 | 0.6329 |
| 2 | 0.6308 |
| 3 | 0.6279 |
| 4 | 0.6297 |
| 5 | 0.6328 |
| 6 | 0.6302 |
| Mean | 0.6307 |
| s | 0.0019 |
| RSD | 0.3025 |

table 15

| Rate pinitol (%) | | | |
|---|---|---|---|
| | day 1 | day 2 | day 3 |
| 1 | 0.6316 | 0.6252 | 0.6360 |
| 2 | 0.6187 | 0.6307 | 0.6292 |
| 3 | 0.6313 | 0.6292 | 0.6412 |
| 4 | 0.6257 | 0.6267 | 0.6378 |
| 5 | 0.6319 | 0.6274 | 0.6386 |
| 6 | 0.6257 | 0.6237 | 0.6441 |
| Mean | 0.6275 | 0.6271 | 0.6378 |
| s | 0.0052 | 0.0026 | 0.0051 |
| RSD% | 0.8264 | 0.4103 | 0.7970 |
| total Mean | 0.6308 | | |
| s total | 0.0066 | | |
| RSD% total | 1.0442 | | |

In terms of reproducibility and intermediate precision, in addition to the same calculations as for the precision of the injection, the 95% confidence interval, the intra-day and inter-day variation are calculated. For this purpose, a unifactorial analysis of variance was performed, i.e., an ANOVA single factor test. For this purpose, it was necessary first to check whether the variances of the groups do not differ significantly from one another, otherwise the ANOVA test may not be used. Table 15 below gives an overview of the D-pinitol content on the various days with mean, standard deviation and relative standard deviation expressed in %.

Looking at this table, it appears that the standard deviations are small and that for day two is smaller, approximately by half, than that of the other days. The coefficients of variance are also relatively small. To determine whether there is a difference between the results for the different days, an ANOVA test was performed. Prior to performing this test it is necessary to determine whether the variances are equal. The Cochran test is used for this, for which the formula (1) is given below:

$$C = \frac{S^2_{max}}{\Sigma_j S^2} \qquad (1)$$

Table 16 below shows a summary of the variances for the various days and calculated and critical Cochran values.

Table 16

| | Variance |
|---|---|
| Day 1 | 0,00002689 |
| Day 2 | 0,00000662 |
| Day 3 | 0,00002584 |
| Critical Cochran value | 0,707 |
| Calculated Cochran value | 0,4530 |

This table shows that the calculated Cochran value is below the critical value. In other words, the variances are considered equal, which means that an ANOVA test can be performed.

Table 17 below gives an overview of the analysis of variance: sum of the squares, degrees of freedom, mean squares and F-values.

Table 17

| *Source of variation* | *Sum of squares* | *Degrees of freedom* | *Mean squares* |
|---|---|---|---|
| Between groups | 0,000440842 | 2 | 0,000220421 |
| Within groups | 0,000296754 | 15 | 1,97836E-05 |

(continued)

| *Source of variation* | *Sum of squares* | *Degrees of freedom* | *Mean squares* |
|---|---|---|---|
| Total | 0,000737595 | 17 | |
| *F* | *P-value* | *Critical range of F-test* | |
| 11,14161558 | 0,001082263 | 3,682316674 | |

The calculated F-value is higher than the critical F-value. From this, it can be concluded that there is a difference between the results on the different days. To determine whether the deviation is still acceptable, the $RSD\%_{between}$ is compared with the 2/3 $RSD\%_{Horwitz}$, which gives an estimate of the maximum RSD% that can be made within a single laboratory. Formula (2) thus considers only the concentration and is as follows:

$$RSD\%_{Horwitz} = 2^{(1-0,5\log C)} \quad \text{where C is the concentration (m/m)} \qquad (2)$$

Table 18 below shows standard deviations, relative standard deviations expressed in per cent, $RSD_{Horwitz}$ and maximum relative standard deviations.

Table 18

| | |
|---|---|
| s within | 0,00445 |
| RSD within | 0,70510 |
| s between | 0,01367 |
| RSD between | 2,16770 |
| RSD Horwitz | 4,28724 |
| RSD max | 2,85816 |

This table shows that the RSD% between is smaller than the RSDmax, from which it can be concluded that the method is still precise despite the fact that differences are seen versus the ANOVA test. Since the RSD% on the results within a single day is very small, the chance is increased that a significant difference will be found with the ANOVA test. Fig. 17 is a graphical representation of the individual measurements and mean values on various days, showing that the measurements overlap.

Table 20

| | Variance |
|---|---|
| 50% | 0,00003907 |
| 100% | 0,00002689 |
| 100% | 0,00000662 |
| 100% | 0,00002584 |
| 200% | 0,00002514 |
| Critical Cochran value | 0,5060 |
| Calculated Cochran value | 0,3162 |

Table 19

Rate pinitol (%)

| | 50% | 100% | 100% | 100% | 200% |
|---|---|---|---|---|---|
| 1 | 0.6374 | 0.6316 | 0.6252 | 0.6360 | 0.6322 |
| 2 | 0.6386 | 0.6187 | 0.6307 | 0.6292 | 0.6416 |
| 3 | 0.6391 | 0.6313 | 0.6292 | 0.6412 | 0.6370 |
| 4 | 0.6462 | 0.6257 | 0.6267 | 0.6378 | 0.6349 |
| 5 | 0.6370 | 0.6319 | 0.6274 | 0.6386 | 0.6452 |
| 6 | 0.6526 | 0.6257 | 0.6237 | 0.6441 | 0.6337 |

(continued)

| Rate pinitol (%) | | | | | |
|---|---|---|---|---|---|
| | 50% | 100% | 100% | 100% | 200% |
| Mean | 0.6418 | 0.6275 | 0.6271 | 0.6378 | 0.6374 |
| s | 0.0063 | 0.0052 | 0.0026 | 0.0051 | 0.0050 |
| RSD% | 0.9739 | 0.8264 | 0.4103 | 0.7970 | 0.7867 |
| total mean | 0.6343 | | | | |
| s total | 0.0076 | | | | |
| RSD% total | 1.1981 | | | | |

The intermediate precision at various concentration levels will be examined in the following. In Table 19 above the contents of D-pinitol in 50 mg, 100 mg and 200 mg samples are shown, with the mean, standard deviation and relative standard deviation for each series. Thus this table 19 gives an overview of the contents of D-pinitol on the various days with mean, standard deviation and relative standard deviation expressed in %.

It is apparent from this table that the errors in the standard deviation for all mean values are in the same order of magnitude everywhere. To determine whether there is a significant difference between the measurements at various concentration levels, here also an ANOVA test was performed after it was confirmed that the variations are not significantly different.

Table 20 above shows a summary of the variances for the different days and calculates a critical Cochran value.

Here also the calculated Cochran value is below the critical value, from which it can be concluded that no significant difference can be demonstrated between the variances of the various groups.

Table 21 below shows an overview of the analysis of variance: sum of squares, degrees of freedom, mean squares and F-values.

Table 21

| *Source of variation* | *Sum of squares* | *Degrees of freedom* | *Mean squares* |
|---|---|---|---|
| Between groups | 0,001057149 | | 0,000264287 |
| Within groups | 0,000617836 | | 2,47134E-05 |
| Total | 0,001674985 | | |

| *F* | *P-value* | *Critical range of F-test* |
|---|---|---|
| 10,69407953 | 342484E-05 | 2,758710593 |

It follows from the ANOVA test in table 21 above that the calculated F-value is greater than the critical F-value and that there is thus a significant difference between the different groups. Graphically, the results at 50% and 200% do not overlap with all results at 100%. Nevertheless, the variation between the groups is still acceptable for the method, since the relative standard deviation expressed in per cent, the coefficient of variance, is less than the $RSD_{max}$, the maximum deviation that may be found in a lab.

Table 22 below shows the standard deviations, relative standard deviations expressed in per cent, $RSD_{Horwitz}$ and maximum relative standard deviations.

Table 22

| | |
|---|---|
| s within | 0,0050 |
| RSD within | 0,7837 |
| s between | 0,0080 |
| RSD between | 1,2675 |
| RSD Horwitz | 2,1418 |
| RSD max | 1,4279 |

Looking at the graph of Fig. 16, it can be concluded that there is no trend in terms of the concentration levels. It can also be concluded from this that sufficient solvent is used and there is no problem with the solubility, otherwise

the values would increase as the number of mg of the sample decreased.

As far as the accuracy is concerned, three types of test setups are possible to find out whether the value measured is also the correct value: the test mixture method, the method of standard additions and comparison with a generally accepted method. Only the method of standard additions can be applied here, since no reconstituted product can be made from the plant material, since not all constituents are known, and there is not yet an acceptable method available, since one needs to be developed. The method of standard additions means that a quantity of sample is added to a known quantity of standard solution. Then a determination is made of how much of the substance is recovered using the following formula:

$$\text{Recovery (\%)} = \frac{X_{after} - X_{before}}{X_{added}} \times 100$$

Table 23 below shows the recovery values with mean, standard deviation, relative standard deviation and 95% confidence interval.

Table 23

| | added (%) | recovery (%) |
|---|---|---|
| 1 | 50 | 99.05483058 |
| 1 | 50 | 101.5438868 |
| 2 | 100 | 107.2875403 |
| **2** | 100 | 105.8382079 |
| 2 | 100 | 105.5938629 |
| 3 | 125 | 106.5970772 |
| 3 | 125 | 105.8311879 |
| 3 | 125 | 106.0000916 |
| Mean | | 104.72 |
| s | | 2.858 |
| RSD | | 2.729 |
| CI | | [102.34 - 107.121 |

Table: 24

| | added (%) | recovery (%) |
|---|---|---|
| 1 | 50 | 101.61 |
| 1 | 50 | 103.65 |
| 1 | 50 | 103.77 |
| 2 | 100. | 103.95 |
| 2 | 100 | 105.69 |
| 2 | 100 | 107.18 |
| 3 | 125 | 104.73 |
| 3 | 125 | 105.26 |
| 3 | 125 | 105.64 |
| Mean | | 104.61 |
| s | | 1.594 |
| RSD | | 1.524 |
| CI | | (103.38 - 105.84] |

The corresponding Fig. 19, is a graphical representation of the values recovered according to the method of standard additions, wherein 1=50% added, 2=100% added, 3=125% added.

Table 24 below shows the recovery values with mean, standard deviation, residual standard deviation and 95% confidence interval.

As is likewise apparent from the corresponding Fig. 20, which illustrates the values found using the method of standard additions, wherein 1=50% added, 2=100% added, 3=125% added, the values in which 50% were added are somewhat lower than when 100% or 125% was added. To determine whether this is simply variability, the experiment must be repeated again. In general, somewhat more than 100% was recovered, which is to be expected when analyses are conducted. It should also be noted that the recovery experiment only shows relative systematic errors and not absolute ones, since the matrix is the same before and after addition.

To determine the specificity, the analysis was repeated with mass spectrometric detection to determine whether only the analyte was measured and no other substances. The analysis was also performed without an internal standard to determine whether no other substances were eluted at the same time. Fig. 21 shows a partial chromatogram of the analysis without internal standard. No interferences were visible at 20 to 21 minutes.

Fig. 22 shows a partial chromatogram of the analysis with internal standard. No interferences were present at the location of xylitol in the chromatogram.

The mass spectroscopic analysis also confirmed that the method is specific. The corresponding mass spectra are shown in the respective Figs. 23 ff, wherein Fig. 23 is a complete chromatogram of the analysis without internal standard, showing the voltage in mV versus minutes.

Fig. 24 shows a complete chromatogram of the analysis of the standards with voltage in mV versus minutes, where the peak at $R_t$ = 20 min is xylitol while the peak at $R_t$ = 22,45 is D-pinitol.

Figs. 25 to 28 show respectively the specificity of a mass spectrum of standard xylitol; xylitol in the sample; D-pinitol sample and standard D-pinitol, showing the relative abundance in per cent as a function of the highest signal.

As far as the *in vivo* evaluation of the hepato-protective effect of *Desmodium adscendens* decoction is concerned, D-pinitol has a hepato-protective effect. One of the constituents of *Desmodium adscendens* is D-pinitol. For the *in vivo* evaluation of the hepato-protective effect of *Desmodium adscendens* decoction it appears from previous analyses that the decoction of the plant originally contains about 0,65% D-pinitol, although this is no longer representative. In this *in vivo* experiment the preventive effect of a decoction of *Desmodium adscendens* against liver injury induced by galactosamine was investigated in rats. Silymarin was used as the reference agent. Silymarin is a mixture of various flavanolignans from the fruits of the milk thistle plant, *Silybum marianum.* The principal components are silybin, silychristine and silydianine. In addition, small quantities of isosilybin are present in milk thistle.

Thus a number of *in vivo* experiments were performed in test animals with the goal of optimising the dose administered and the treatment schedule and looking for a possible effect on liver injury by ethanol and acetaminophen.

In the experiment performed, the following scheme was used for six groups of test animals:

*Desmodium* decoct 20 mg/kg;
*Desmodium* decoct 5 mg/kg;
D-pinitol 20 mg/kg;
Silymarin 20 mg/kg (positive control);
No treatment, galactosamine intoxication with single dose 650 mg / kg (negative control);
No treatment, no intoxication.

The treatment schedule was as follows:

- Day 0: for treatment with *Desmodium* decoct: 20 mg / kg or 5 mg / kg

    pretreatment with D-pinitol: 20 mg / kg
    pre-treatment with silymarin: 20 mg / kg

- Day 1: pre-treatment with *Desmodium* decoct: 20 mg / kg or 5 mg / kg

    pretreatment with D-pinitol: 20 mg / kg
    pre-treatment with silymarin: 20 mg / kg
    immediately followed by administration gelactosamine

- Day 2: all blood groups (24 h)

    treatment with *Desmodium* decoct: 20 mg / kg or 5 mg / kg
    Treatment with D-pinitol: 20 mg / kg
    treatment with silymarin: 20 mg / kg

- Day 3: all blood groups (48 h).

The following 3 control groups had to be present in each experiment:

- Silymarin 20 mg / kg (positive control);
- no treatment, only galactosamine intoxication 650 mg/kg (negative control);
- no treatment, no intoxication.

In the experiment performed, the effect after 24 h did not yet seem very pronounced, but it was after 48 h. Therefore, no blood samples were taken at 24 h in the follow-up experiments, but only after 48 h, followed by a second sample after at least an additional 24 h, and possibly a third blood sample at an even later time.
Optimisation of the dose and treatment schedule was performed in experiments with D-pinitol. Then this dose was calculated based on *Desmodium* decoction for another experiment.
To determine the hepato-protective effect of the preparation being tested, therefore, the selected experimental animals, namely Sprague Dawley rats, underwent pre-treatment on day 0 and day 1 before the hepatotoxic agent was administered (day 1). On day 2 there was also a single aftertreatment. The *Desmodium* decoction, the pure active ingredient D-pinitol, and the positive control silymarin were all administered orally by gavage.
This resulted in the aforementioned 6 test groups, namely:

| | |
|---|---|
| Control | No hepatotoxic agent (vehicle), no treatment (vehicle) |
| Hepatotox: | Hepatotoxic effect, no treatment (vehicle) |
| Desmodium 1: | Hepatotoxic effect, treatment with *Desmodium* equivalent to 20 mg/kg D-pinitol |
| Desmodium 2: | Hepatotoxic effect, treatment with *Desmodium* equivalent to 5 mg/kg D-pinitol |
| D-pinitol: | Hepatotoxic effect, treatment with pure D-pinitol 20 mg/kg |
| Silymarin: | Hepatotoxic effect, treatment with silymarin 20 mg/kg. |

In summary, the treatment schedule was as follows: on day 0 and day 1 a pre-treatment was given with one of the test preparations. After the pre-treatment, the groups of experimental animals in question were given a hepatotoxic product on day 1: D-galactosamine 650 mg/kg IP 2% in physiol. solution. On day 2 a treatment dose was likewise administered. On day 2 and day 3, 1,5 ml blood was collected from the tail vein. The liver injury and the possible hepato-protective effect were evaluated by determining the following parameters in serum: ALT (=GPT), AST (=GOT), ALP.
The results of the tests were as follows; see the respective tables and Figs. 29 ff. After administration of the hepatotoxic agent D-galactosamine, both after 24 h and after 48 h, liver injury was observed through a marked increase in the three parameters determined (control vs. hepatotox).
After 24 h, no significant decrease in AST (GOT) and ALP was seen for any treatment compared with the hepatotoxic group, thus also not for the positive control silymarin. A significant decrease was seen in ALT (GPT) in both *Desmodium* groups and with silymarin.
After 48 hours, the results were more pronounced: both AST (GOT) and ALT (GPT) had decreased significantly compared with the hepatotoxic group for all treatments: both *Desmodium* dosages, D-pinitol and silymarin. A few noteworthy observations were that the lowest *Desmodium* dosage (equivalent to 5 mg/kg D-pinitol) already has a very pronounced effect, which is comparable to the highest dose (equivalent to 20 mg/kg D-pinitol); that both *Desmodium* doses are more active than, or at least as active as, silymarin; and that both *Desmodium* doses are more active than or at least as active as 20 mg/kg pure D-pinitol.
As far as the parameter ALP is concerned, after the treatment was administered, no effect could yet be seen. Perhaps longer treatment is required for this.
Thus it can be concluded that the hepato-protective effect of the standardised *Desmodium adscendens* preparation is clearly demonstrated in the treatment schedule used. Further study on the dose-dependence of the effect is indicated, in view of the good results obtained with the lowest dose of *Desmodium* decoction. The effect of longer pre-treatment, of a therapeutic treatment that only starts after administration of the hepatotoxic agent, and a combination of the two can be further investigated, possibly on a broader set of liver injury parameters.
Another experiment consisted of the design and execution of an experiment with ethanol-induced liver injury instead of galactosamine, with the same doses of *Desmodium* decoction as in the previous experiment.
The anti-hepatotoxic activity of a standardised *Desmodium adscendens* decoction and D-pinitol against chemically induced liver injury in rats was investigated, in particular the protective effect of *D. adscendens* decoction against ethanol-induced liver injury.
The purpose of the experiment is to evaluate the hepatoprotective effects of decoct of *Desmodium adscendens* against ethanol-induced liver injury, standardized to its primary ingredient: D-pinitol. Materials and Methods: 66 male Wistar rats of 200-225g (Charles River, Brussels, Belgium) were randomly divided into 6 groups: the negative control group (CON: no hepatotoxic agent, no treatment: 8 rats), the hepatotoxic group (HEP : induction of hepatotoxicity, no

treatment: 20 rats), the *Desmodium* I group (induction of hepatotoxicity, treatment with *Desmodium adscendens* decoct, equivalent to 2 mg / kg of D-pinitol: 10 rats), the group II *Desmodium* (induction of hepatotoxicity, treatment with *Desmodium adscendens* decoct, equivalent to 10 mg / kg of D-pinitol: 10 rats), the D-pinitol group (PIN: induction of hepatotoxicity, treatment with 10 mg/kg of D-pinitol: 10 rats), the positive control group (SIL: induction of hepatotoxicity, treatment with 20 mg/kg of silymarin: 8 rats). 2 days prior to ethanol administration, an appropriate amount of lyophilized decoct, pinitol silymarin or suspended in water and administered by gavage (or vehicle for the control group). Hepatotoxicity was induced by daily oral gavage with a 55% ethanol solution (except for the negative control group). An initial dose of 2g/kg of ethanol was gradually increased to a dose of 6g/kg during the first week of the experiment. Ethanol was administered for a period of 7 weeks. Was administered daily, depending on the group, treated with the specific decoct, pinitol or silymarin (oral gavage) to the end of the experiment. For ethanol administration (default values), and at weeks 3, 4, 5 and 6, blood samples (1,5 ml) were taken from the lateral tail vein (Multi Fat 600, Sarstedt). This experiment was approved by the Ethics Committee for Animal Experiments of the University of Antwerpen (17-01-2011, 2010-37).

| |
|---|
| CON: no ethanol, no treatment |
| HEP: ethanol, no treatment |
| DES 1: ethanol, *Desmodium adscendens* treatment equivalent to 2 mg / kg of D-pinitol |
| DES 2: ethanol, *Desmodium adscendens* treatment equivalent to 10 mg / kg of D-pinitol |
| PIN: ethanol, D-pinitol treatment (10 mg / kg) |
| SIL: ethanol, silymarin treatment (20 mg / kg) |

A schematic overview of the different treatment groups is given below next. Concentrations of enzyme aspartate transaminase (AST, GOT) and alanine transaminase (ALT, GPT) were in the serum samples from the test animals as determined by routine laboratory techniques (Senior, 2009). Elevated levels can be considered as an indication of liver cell destruction or a change in membrane permeability.

Statistics to analyze the difference between CON and HEP for AST and ALT, and between HEP and DES 1, DES 2, PIN and SIL for AST and ALT, was a mixed model analyze performed. The log-rank test was performed for statistical analysis of survival data.

Results: Serum AST and ALT values in weeks 4, 5 and 6 of the study are shown in Tables 26-31, and Fig. 32-34, 36-38. Average AST and ALT values at different points in time (weeks 0, 2-6) are shown in Fig. 35 and 39. Tables 32 and 33 and Fig. 40 show the survival time in the different animal groups.

Serum AST

Table 27

| | Average (U/I) | AST_5 (U/I) | SEM (U/I) |
|---|---|---|---|
| CON | 65.3 | 7.4 | 2.5 |
| DES1 | 75.4 | 8.6 | 3.5 |
| DES2 | 75.5 | 11.5 | 4.7 |
| PIN | 78.3 | 15.8 | 7.1 |
| SIL | 72.1 | 8.6 | 5.0 |
| HEP | 83.8 | 8.7 | 2.7 |
| | | ↓ | |
| | | Standard-deviation | |

Table 26

| | Average (U/I) | AST_4 (U/I) | SEM (U/I) |
|---|---|---|---|
| CON | 63,6 | 6,7 | 2,4 |
| DES1 | 79,8 | 12,3 | 5,5 |
| DES2 | 77,3 | 9,9 | 3,7 |
| PIN | 89,3 | 9,3 | 3,8 |
| SIL | 83,2 | 21,7 | 9,7 |

(continued)

| | Average (U/l) | AST_4 (U/l) | SEM (U/l) |
|---|---|---|---|
| HEP | 84,2 | 16,3 | 5,1 |
| | | ↓<br>Standard-deviation | |

Table 26 above right shows Serum AST values after 4 weeks of ethanol administration. Fig. 32 shows Serum AST values after 4 weeks of ethanol administration, * p<0,05, ** p<0,01, *** p<0,001 vs HEP CON; † p<0,05 vs HEP DES 1, DES2, PIN, SIL (Mixed model analysis).

Table 27 above left shows Serum AST values after 5 weeks of ethanol administration.

Fig. 33 shows Serum AST values after 5 weeks of ethanol administration, * p<0,05, ** p<0,01, *** p<0,001 vs HEP CON; † p<0,05 vs HEP DES 1, DES2, PIN, SIL (Mixed model analysis).

Table 28 below right shows serum AST levels after 6 weeks of ethanol administration.

| Serum | ALT | ALT_4 | |
|---|---|---|---|
| Average | Standard | Deviation | SEM |
| CON | 43.6 | 9.1 | 3.0 |
| DES1 | 71.0 | 29.1 | 11.9 |
| DES2 | 58.8 | 14.0 | 5.3 |
| PIN | 75.9 | 15.5 | 6.3 |
| SIL | 57.4 | 9.9 | 4.4 |
| HEP | 63.2 | 19.0 | 5.7 |

| | | AST_6 | |
|---|---|---|---|
| | Mean (U/l) | Standard deviation (U/l) | SEM (U/l) |
| CON | 53.1 | 6.8 | 3.0 |
| DES1 | 75.9 | 15.3 | 6.3 |
| DES2 | 75.9 | 13.2 | 5.4 |
| PIN | 86.3 | 9.4 | 4.7 |
| SIL | 77.8 | ,3 | ,2 |
| HEP | 75.0 | 4.1 | 2.0 |

Fig. 34 shows serum AST levels after 6 weeks of ethanol administration, * p <0,05, ** p <0,01, *** p<0,001 vs HEP CON; † p<0,05 vs HEP DES 1, DES2, PIN, SIL (Mixed·model analysis). Fig. 35 shows AST Average values per time point (weeks 0,2,3,4,5,6).

Table 29 above shows Serum ALT levels after 4 weeks of ethanol administration.

| | | ALT_6 | |
|---|---|---|---|
| Mean/Standard. | Deviation/Standard. | Error.of.Mean | |
| CON | 37.0 | 9.1 | 4.1 |
| DES1 | 56.6 | 7.7 | 3.1 |
| DES2 | 50.1 | 12.2 | 5.0 |
| PIN | 65.6 | 15.7 | 7.8 |
| SIL | 59.7 | 9.3 | 6.6 |
| HEP | 47.9 | 5.3 | 2.6 |

| | | ALT_5 | |
|---|---|---|---|
| Mean/Standard. | Deviation/Standard. | Error. of. Mean | |
| CON | 39.8 | 7.6 | 2.5 |

(continued)

| ALT_5 | | | |
|---|---|---|---|
| Mean/Standard. | Deviation/Standard. | Error. of. Mean | |
| DES1 | 52.3 | 10.7 | 4.4 |
| DES2 | 52.8 | 17.5 | 6.6 |
| PIN | 61.6 | 19.2 | 8.6 |
| SIL | 44.8 | 6.9 | 4.0 |
| HEP | 52.0 | 13.4 | 4.2 |

Table 30 above shows Serum ALT levels after 5 weeks of ethanol administration.
Fig. 36 shows serum ALT values after 4 weeks of ethanol administration, * p <0,05, ** p <0,01, *** p<0,001 vs HEP CON; † p<0,05 vs HEP DES 1, DES2, PIN, SIL (Mixed model analysis).
Fig. 37 shows serum ALT levels after 5 weeks of ethanol administration, * p<0,05, ** p<0,01, *** p<0,001 vs HEP CON; † p<0,05 vs HEP DES 1, DES2, PIN, SIL (Mixed model analysis).
Table 31 above shows serum ALT levels after 6 weeks of ethanol administration.
Fig. * P<0,05; ** p<0,01, Figure 38 shows serum ALT levels after 6 weeks of ethanol administration, *** p<0,001 vs HEP CON; † p<0,05 vs HEP DES 1, DES2, PIN, SIL (Mixed model analysis).
Fig. 39 shows ALT Average values per time point (weeks 0,2,3,4,5,6).

Mortality

Table 32 shows the survival of test animals in the test groups over a period of 6 weeks.

| | CON | DES1 | DES2 | PIN | SIL | HEP |
|---|---|---|---|---|---|---|
| WO | 100% | 100% | 100% | 100% | 100% | 100% |
| W1 | 100% | 100% | 100% | 100% | 100% | 95% |
| W2 | 100% | 90% | 100% | 70% | 78% | 85% |
| W3 | 100% | 70% | 80% | 60% | 56% | 55% |
| W4 | 100% | 60% | 70% | 60% | 56% | 40% |
| W5 | 100% | 60% | 70% | 50% | 33% | 40% |
| W6 | 100% | 60% | 60% | 40% | 22% | 25% |

Fig. 40 shows the survival of test animals in the test groups over a period of 6 weeks. Table 33 shows log-rank test for the determination of difference in the survival of the animals over a period of 6 weeks.

| Comparison | P-value |
|---|---|
| CON - HEP | 0.0044 |
| HEP-DES1 | 0.11 |
| HEP-DES2 | 0.06 |
| HEP-PIN | 0.56 |
| HEP-SIL | 0.88 |
| All treated groups | 0.141 |

Table 33

In this experiment, wherein the preventive effect of *Desmodium adscendens* decoct against ethanol-induced liver injury was evaluated, the hepatotoxic group (HEP) shows significantly increased serum levels of AST and ALT after

4 weeks of daily ethanol administration (Fig. 32-34, 36-38). Treatment with *Desmodium adscendens* (2 mg/kg and 10 mg/kg), pinitol (10 mg/kg) or silymarin (20mg/kg) was started 2 days before administration of ethanol and further put daily until the end of the experiment, 6 weeks later. As shown in Fig. 32-34 and 36-38, no significant reduction was observed with regard to serum AST (fig. 32, 33 & 34 after treatment of resp. 4, 5 & 6 weeks), and ALT levels (fig. 36, 37 & 38 after treatment of resp. 4, 5 & 6 weeks), for any treatment. Evaluation of the mortality of the animals in the different treatment groups (Fig. 40, Table 32), showed a large drop in the untreated group hepatotoxic (HEP), while survival was better in DES 2. Moreover, after 6 weeks of ethanol-administration, 60% of the rats that were given decoct *Desmodium adscendens* survived, while the survival rate in the pinitol and silymarin-treated groups was 40%, and respectively 22%. Statistical analysis of the survival data (Table 33) shows a statistically significant difference in survival between control and hepatotoxic group ($p<0,01$), and a trend towards significance ($p=0,06$) between the *D. adscendens* group DES 2 (eq. to 10 mg/kg pinitol) and the untreated group hepatotoxic. No statistically significant difference between the group DES 1 (eq. to 2 mg/kg pinitol), pinitol group (10 mg/kg) group or silymarin (20 mg/kg) and the hepatotoxic group was observed.

Since a period of at least 4 weeks was needed to produce significant ethanol-induced hepatotoxic effects, in rats, and a large drop of the hepatotoxic untreated animals was observed, it was not possible to investigate the hepato-curative effects of *Desmodium adscendens* decoct in this experiment.

A further experiment consisted in setting up and conducting an experiment with acetaminophen-induced liver damage instead of galactosamine.

The experiment aimed to evaluate the hepato-curative effects of a decoction of *Desmodium adscendens,* standardized on its main component D-pinitol, against acetaminophen (paracetamol)-induced liver injury.

The materials and methods used for this purpose consisted of 40 male Wistar rats of 200-225g, which were randomly divided into 5 groups: the negative control group (CON: no hepatotoxic agent, no treatment: 8 rats), and the hepatotoxic group (HEP: induction of hepatotoxicity, no treatment: 8 rats), and the group I *Desmodium* (induction of hepatotoxicity, treatment with *D. adscendens* decoct, equivalent to 2 mg/kg of D-pinitol: 8 rats), and the group II *Desmodium* (induction of hepatotoxicity, treatment with *D. adscendens* decoct, equivalent to 10 mg/kg of D-pinitol: 8 rats), and the positive control group (SIL: induction of hepatotoxicity, treatment with 20mg/kg of silymarin: 8 rats). Acute hepatotoxicity was induced by oral gavage with 2 g/kg acetaminophen (except the control group). 24 h after hepatotoxicity induction, treatment was initiated with the appropriate amount (see above), lyophilized extract of *D. adscendens* or silymarin, suspended in water and administered by gavage (or vehicle for the control groups and hepatotoxic). Blood samples were taken from the lateral take-off vein (1,5 ml, Multi Fat 600, Sarstedt) 24h, 48h, and 72h after acetaminophen administration. This experiment was approved by the Ethics Committee for Animal Experiments of the University Antwerpen (17-01-2011, 2010-37).

A schematic overview of the different treatment groups is shown below:

CON: no acetaminophen, no treatment; HEP: 2g/kg acetaminophen, no treatment DES 1: 2g/kg acetaminophen, *D. adscendens* treatment equivalent to 2 mg/kg of D-pinitol

DES 2: 2g/kg acetaminophen, *Desmodium adscendens* treatment equivalent to 10 mg/kg D-pinitol SIL: 2g/kg acetaminophen, silymarin treatment (20 mg/kg).

Enzyme levels of aspartate transaminase (AST, GOT) and alanine transaminase (ALT, GPT) were determined in serum samples from animals with routine laboratory techniques (Senior, 2009).

Increased levels can be regarded as an indication of liver cell destruction or a change in membrane permeability. For statistical purpose, a One way ANOVA was performed, so as to analyze the difference between CON and HEP for AST and ALT, and between HEP and DES 1, DES 2 and SIL for AST and ALT, which was followed by Dunnett post hoc test (SPSS statistics program). The results for serum AST and ALT values at 48h and 72h after acetaminophen administration are shown in Tables 34-37, and Fig. 29-31.

Table 34

| | AST 48 | | |
|---|---|---|---|
| | mean (U/l) | Standard Deviation (U/l) | SEM (U/l) |
| CON | 62 | 9 | 4 |
| DES1 | 403 | 363 | 128 |
| DES2 | 717 | 753 | 266 |
| SIL | 1047 | 1155 | 408 |
| HEP | 704 | 673 | 213 |

Table 35

| | AST 72 | | |
|---|---|---|---|
| | mean (U/l) | Standard Deviation (U/l) | SEM (U/l) |
| CON | 62 | 10 | 3 |
| DES1 | 76 | .19 | 7 |
| DES2 | 99 | 38 | 13 |
| SIL | 110 | 47 | 17 |
| HEP | 81 | 12 | 4 |

Fig. 29 shows Serum AST values 48h after acetaminophen administration, * p<0,05, ** p<0,01, *** p<0,001 vs. CON HEP (Statistics: One-Way ANOVA, post hoc Dunnett versus HEP). Serum ALT

Table 36 & 37 below show Serum ALT values 48 and 72h after acetaminophen administration.

| | alt48 | | |
|---|---|---|---|
| | Gemiddel de (U/l) | Standaard Deviatie (U/l) | SEM(U/l) |
| CON | 28 | 6 | 2 |
| DES1 | 208 | 161 | 57 |
| DES2 | 451 | 483 | 171 |
| SIL | 423 | 380 | 134 |
| HEP | 327 | 295 | 93 |

| | alt72 | | |
|---|---|---|---|
| | Gemiddel de (U/l) | Standaard Deviatie (U/l) | SEM (U/l) |
| CON | 36 | 16 | 6 |
| DES1 | 49 | 18 | 6 |
| DES2 | 79 | 52 | 18 |
| SIL | 70 | 42 | 15 |
| HEP | 59 | 13 | 5 |

Fig. 30, 31 show Serum ALT values 48 and 72h after acetaminophen administration, * p<0,05, ** p<0,01, *** p<0,001 vs. CON HEP (Statistics: One-Way ANOVA, Dunnett post hoc versus HEP). In this experiment, that evaluated the curative effect of *Desmodium adscendens* decoct against acetaminophen-induced liver damage, the hepatotoxic group (HEP) showed significantly increased levels of serum AST and ALT at 24h and 48h after acetaminophen administration (p<0,001), and also at 72h after acetaminophen administration for ALT (p<0,05). An oral treatment of *Desmodium adscendens* (2 mg/kg and 10 mg/kg) or silymarin (20 mg/kg, positive control group) was given 24h and 48h after acetaminophen administration. As shown in Fig. 29-31 no significant decrease (p>0,05) could be observed for a serum AST and ALT levels in blood sampling points 48h and 72h for any treatment. The results of this experiment in a rat model of acute hepatic damage, induced by acetaminophen, show that *Desmodium adscendens* has no hepatocurative effect when administered in a daily dose of up to 10 mg/kg.

Finally, a so-called Ames test was performed on samples of UA: *Desmodium* extract. The extract was tested according to the OECD guideline with 5 *Salmonella typhimurium* strains (TA98, TA100, TA102, TA1535 and TA1537) in the absence and presence of a metabolising S9 fraction. For this experiment, fresh strains were used, also S9 and other needed materials such as NADPH, Petri dishes, etc.

In accordance with the guidelines, the tests were started with a maximum test concentration of 5 mg/plate, after which dilutions were carried out using 6 dosages. A negative (solvent) control was used with 4 Petri dishes, as well as a positive control with 3 Petri dishes. Positive controls are part of the recommended list of controls at the recommended test concentration. A list of the positive controls that we used and their concentrations is available.

Thus three Petri dishes were prepared for each concentration and 4 for the negative control. The results showed the mean values thereof, in particular the mean number of revertants ± SD. The test is successful if the negative and

positive concentrations are within the expected range and no signs of toxicity are seen. This was determined by examining the background layer of bacteria, wherein the absence of a background layer indicates toxicity. No signs of toxicity were found, and all strains and controls gave the expected response to TA102. A test substance is positively "mutagenic" if a doubling of the number of revertants is seen compared with the negative (solvent) control, and if a dose-effect relationship is observed.

A synthesis of the results is presented in the following. The above data indicate that the sample is not mutagenic in the strains TA98, TA100 and TA1535, both in the presence and in the absence of S9. For TA1537, just a doubling of the mutation frequency was found in the presence of S9 at the highest concentration, 5 mg/plate, relative to the negative control, but no pronounced dose-effect relationship. To be certain, the test was repeated. No dose-effect relationship was found, and also no doubling with respect to the control, which confirms that there was also no mutagenic effect in strain TA1537.

It could be established experimentally that there is practically no mutagenic effect. The *Desmodium* extract is not mutagenic in the Ames test either in the absence or in the presence of a metabolising S9 fraction. An increase was seen in the number of spontaneous revertants at the highest dose, but this was nevertheless insufficiently great to use the term "mutagenicity." Repeated experiments (at least 1 per strain) confirm the absence of mutagenicity.

Regarding flavonoid content and profile in *Desmodium adscendens* lyophilisate,

In the employed method, there is firstly the sample preparation:

To prepare the standard (reference solution), 5,0 mg vitexin was weighed into a 10,0 ml volumetric flask and dissolved in methanol. 3 ml of this were pipetted over to a 20,0 ml volumetric flask and this was diluted with 50% methanol.

For preparing the samples (test solutions), in each case about 100 mg powder (lyophilisate) is weighed into a 25,0 ml volumetric flask and 20 ml 50% methanol is added. The solutions are then sonicated for 15 minutes in the ultrasonic bath. After the samples are cooled, they are diluted to 25,0 ml with 50% methanol. Both the test solution(s) and the reference solution(s) are filtered through a nylon syringe filter.

HPLC conditions

The mobile phases used were A: 1,0% (v/v) phosphoric acid in water and B: acetonitrile. The gradient conditions were as follows:

Start conditions: 5% B
0-5 min.→ 10% B 5-80 min.→ 50% B 85-90 min.→ 5% B

With a flow of 1,0 ml/min. Detection is at 360 nm. Column used is a Grace smart column or Lichrosphere column (250 x 4,5 microns).

Results: Total content of flavonoids as vitexin in a typical lyophilisate of *D. adscendens,* wherein all peaks with a UV spectrum typical of flavonoids are totalled: 1,122%

Typical HPLC profile

The indicated peaks show a UV spectrum characteristic for flavonoids.

*Desmodium adscendens* lyophilisate

Expansion to flavonoids with chromatographic profile and content

Method used

Sample preparation see above.

HPLC conditions'

As mobile phase, A: H2O with 0,1% formic acid and B: acetonitrile are used. The gradient conditions are as follows:

Start conditions: 15% B
0-5 min →15% B 5-39 min →23% B ◊ ◊ 39-43 min →100% B
43-45 min →100% B ◊ 45-47 min →15% B 47-55 min →15% B

with a flow rate of 1,0 ml / min. Detection is at 334 nm (DAD). As a column, a Luna C18 column (Phenomenex) (250 x 4 mm, 5 um) is used.

Results

Flavonoid profile + identification

With the indicated method, a chromatographic profile was obtained as shown in the Figure. Diode Array Detection (DAD) of the labelled signals (A-F) led to the UV spectra shown. All labelled signals (peak A-peak F) exhibit a UV

spectrum that is characteristic of flavonoids.

After isolation of the products involved, the principal component (peak E) was identified as vitexin-2"-xyloside on the basis of mass spectrometry and NMR spectroscopy. The table shows the assignments of the $^{1}$H and $^{13}$C-NMR spectra compared with published data. In this way it was also possible to identify peak F spectroscopically as vitexin.

Content determination

The total content of flavonoids, determined as the sum of the labelled signals, expressed as vitexin, in a typical lyophilisate of (the decoction of) *D. adscendens* was 1.05%.

Table

$^{1}$H and $^{13}$C-NMR assignments for peak E (taken up in DMSO-$d_6$) and vitexin-2"-xyloside
13 C (ppm) 1 H (ppm) 13 C (ppm) 1 H (ppm)
2 163.8 164.2
3 102.5 6.70 102.8 6.80 s
4 181.6 182.4
5 160.6 13.10 (OH) 160.9
6 99.0 6.18 98.5 6.27 s
7 162.5 163.1
8 104.1 104.0
9156 156.9
10 103.3 104.1
1 ’121.7 121.9
2 ’, 6’ 128.9 7.99 (d, 8,4 Hz) 8:04 129.2 (d, 8,6 Hz)
3 ’, 5’ 116.3 6.89 d, 8,4 Hz) 116.3 6.94 (d, 8,6 Hz)
4 ’161.7 161.5
Glc-1 71.9 4.79 (d, 71.9 4.83 (d, 9,8 Hz)
Glc-2 81.7 3.24 82.1 3.32 m
Glc-3 78.8 3.46 78.6 3.58
Glc-4 70.5 3.42 70.5 3.54
Glc-5 80.8 4.06 81.6 4.09 brt
Glc-6 61.2 3.73 61.3 3.79 3:54, 3.60
Xyl-1 106.2 3.88 106.1 3.91 (d, 7,0 Hz)
Xyl-2 73.0 2.75 74.0 2.80 dd
Xyl-3 76.0 2.84 76.2 2.90 br t
Xyl-4 69.0 3.07 69.7 3.02
Xyl-5 65.7 3.07,2.33 65.8 3.02, 2:39

## Claims

**1.** Method for producing a plant extract, quantified on D-pinitol, wherein a plant *Desmodium adscendens* is selected from the *Desmodium* family, wherein a fraction is extracted from *Desmodium* plant parts, wherein a plant extract is derived from said fraction thereof,
**characterized in that** a characterised extract is derived from *Desmodium adscendens,* from which a preparation of said plant extract is quantified on D-pinitol;
wherein said plant extract contains between 0,1 and 10%, preferably from 4 to 5% D-pinitol; particularly wherein a validated analytical method is performed for said quantification of *Desmodium adscendens* on D-pinitol, wherein a specific quantified *Desmodium adscendens* preparation is produced as *"totum",* more particularly wherein a biologically controlled isolation is carried out in said analytical method which is carried out with generating a composition quantified for D-pinitol, wherein the composition product is repeatedly enriched and numerous fractions are isolated, and wherein a decoction is obtained that contains a significant amount of D-pinitol;
wherein said extract is separated and the isolated product is identified as the methylated cyclitol 3-O-methyl-chiro-inositol, i.e. D-pinitol,
**in that** selected parts of said plant are first dried, and/or **in that** said plant parts are then ground or fragmented,

particularly pulverized into powder form, more particularly wherein said powder is converted as such into a pharmaceutical form; wherein starting from 1 kg of dried plant parts, there is obtained from 60 to 70 g, in particular about 65 g of extract, respectively according to a ratio of 10-20 : 1, more particularly 14-16 : 1;
**in that** an aqueous decoction of said plant parts of *Desmodium_adscendens* is then prepared warm to a decoct by boiling a certain quantity of dried, optionally powdered, leaves in a certain quantity of water for a certain period of time and **in that** said aqueous decoction of said plant parts of *Desmodium adscendens* is then cooled, after which the portions obtained are combined and filtered, after which the filtrate obtained is concentrated, in particular under vacuum, and then lyophilized to form a lyophilizate.

**2.** Method according to the preceding claim, **characterized in that** the constituents of said composition are standardised with capture of the reproducibility of the production process,
and/or **in that** the main components of the constituents in *Desmodium adscendens* and the total amount of D-pinitol are determined by means of an analytical method therefor, wherein the extraction process, the products obtained after extraction and the conditions are evaluated and optimised.

**3.** Method according to any one of the preceding claims for producing a plant extract enriched in D-pinitol, **characterized in that** it comprises the steps of

i) extracting parts of a plant of *Desmodium adscendens* with water, with a lower alkyl alcohol, notably including methanol, ethanol and/or isopropanol, or combinations thereof, or with a mixture of one or more lower alkyl alcohols, or with a mixture of water and one or more lower alkyl alcohols, with a mixture of water, one or more lower alkyl alcohols and one or more less polar or non-polar organic solvents, or carbon dioxide, to obtain a decoction comprising pinitol, and
ii) filtering and concentrating said decoction;
particularly wherein it further comprises the steps of
iii) separating the decoction by column chromatography to obtain several fractions; and
iv) combining those fractions.

**4.** Method according to any one of the preceding claims, **characterized in that** an aqueous decoction of said plant parts of *Desmodium adscendens* is then prepared warm to a decoct by boiling a certain quantity of dried, optionally powdered, selected parts of said plant consisting of leaves in a certain quantity of water or distilled water, for a certain period of time; particularly wherein said aqueous decoction of said plant parts of *Desmodium adscendens* is then cooled, after which the portions obtained are combined and filtered, after which the filtrate obtained is concentrated, and then lyophilized to form a lyophilizate; or after which the resulting portions are pooled and filtered, after which the filtrate is concentrated, and then spray-dried; in particular wherein the filtrate is resp. concentrated under vacuum.

**5.** Method according to any one of the claims 1 to 4, **characterized in that** an aqueous product as macerate of said plant parts of *Desmodium adscendens* is then cold-processed to an extract, in particular by incorporating a certain amount of dried, optionally pulverized plant parts of to be included in a certain amount of water or distilled water, for a certain period of time.

**6.** Method according to any one of the claims 3 to 5, **characterized in that** the chromatography is performed over a Sephadex LH-20 as gel filtration medium having a 4000-5000 dalton exclusion limit, wherein the elution of the chromatography is performed with methanol.

**7.** Method according to one of the claims 3 to 6, **characterized in that** said fractions are combined into a number of sub-fractions, in particular 11, according to their chromatographic pattern, particularly wherein the fractions combined are those that react with 1 % anisaldehyde in sulfuric acid to yield a product having a green color;
wherein said sub-fractions 5-11, in particular 200 mg, which have a spot with a green color, are combined, after spraying with anisaldehyde 1% $H_2SO_4$ in MeOH and heating to about 120°C for 10 min, wherein said fraction is subjected to further column chromatography eluted with MeOH, wherein certain fractions, in particular of 100 ml, are once again collected and analysed,
wherein said sub-fractions 4-5, in particular 120 mg, from this column are combined, wherein the latter are subjected to a further column chromatography under the same conditions, after which a pure product is obtained, in particular white;
more particularly further comprising the steps of
v) separating the combined fractions by gel filtration chromatography; yet more particularly wherein the chromatography is performed over a Sephadex LH-20 as gel filtration medium having a 4000-5000 dalton exclusion limit and the elution is performed with methanol.

**8.** Method according to any one of the preceding claims, **characterized by** an extension of the characterization of the *Desmodium adscendens* preparation with a flavonoid profile, particularly wherein vitexin is identified wherein the total amount of flavonoids, defined as the sum of the signals obtained, expressed as vitexin, amounts to approximately 1,05%, in a typical lyophilizate of the decoct of *Desmodium adscendens,* as flavonoid rate and profile in *Desmodium adscendens* lyophilisate between 0,1 and 5%, more particularly wherein said rate is of the order of 1%.

**9.** Method according to any one of the preceding claims, particularly 3 to 8, more particularly 7, for purifying D-pinitol from a plant, **characterized in that** it comprises:

i) extracting parts of a plant of *Desmodium adscendens* with water, with a lower alkyl alcohol, with a mixture of one or more lower alkyl alcohols, or with a mixture of water and one or more lower alkyl alcohols, with a mixture of water, or carbon dioxide, to obtain a decoction comprising pinitol, and
ii) filtering and concentrating said decoction;
iii) separating the decoction by chromatography to obtain several fractions;
iv) combining those fractions that react with 1 % anisaldehyde in sulfuric acid to yield a product having a green color; wherein
the sub-fractions 5-11, in particular 200 mg, which have a spot with a green color, are combined, after spraying with anisaldehyde 1% H2SO4 in MeOH and heating to about 120°C for 10 min, wherein said fraction is subjected to further column chromatography eluted with MeOH, wherein certain fractions, in particular of 100 ml, are once again collected and analysed;
v) separating the pinitol-containing fraction by gel filtration chromatography; in particular white, particularly wherein the chromatography steps are performed using a Sephadex LH-20 as medium with a 4000-5000 dalton exclusion limit and elution is performed with methanol; more particularly wherein the product is D-pinitol;
wherein the sub-fractions 4-5, in particular 120 mg, from this column are combined, wherein the latter are subjected to a further column chromatography under the same conditions, after which a pure product is obtained, in particular white; and
wherein said extract is separated and the isolated product is identified as the methylated cyclitol 3-O-methyl-chiro-inositol, i.e. D-pinitol.

**10.** Method according to one of the claims 3 to 9, **characterized in that** sugar alcohols are selected as an internal standard, in particular xylitol, notably in gas chromatography, or possibly others such as sorbitol, mannitol, optionally dulcitol and inositol, instead of the abovementioned xylitol, with the latter two in a lesser extent.

**11.** Method according to any one of the claims 1 to 10, **characterized in that** a standardized extract is derived from the plant *Desmodium adscendens,* quantified for D-pinitol in its action against hepatitis, in particular in preventive or possibly curative action, with the isolation of D-pinitol from *Desmodium adscendens,* wherein D-pinitol forms an active ingredient, particularly with regard to liver damage of chemical, physical, infectious or immunological origin.

**12.** Plant extract obtained by a method as defined in one of the preceding claims for protecting the liver in a mammal, for the prevention of a liver disease in a mammal and/or for treatment thereof; particularly wherein it is derived from *Desmodium adscendens* quantified on the molecule D-pinitol active against hepatitis, wherein D-pinitol is isolated from *Desmodium adscendens* on an active ingredient, standardized forms; more particularly wherein said plant extract contains between 0,1 and 10%, preferably from 4 to 5% D-pinitol.

**13.** A pharmaceutical composition comprising the extract of claim 12.

**14.** Composition for use as a medicament comprising a vegetable extract as defined in claim 13, particularly **characterized in that** this is employed as an anti-oxidizing agent for specific target groups, in particular where the target referred formed by man, more particularly alcoholics and/or by patients with a metabolic syndrome, yet more particularly by diabetes patients with type 2 diabetes, particularly for treating hepatitis, diabetes or metabolic syndrome, or for obtaining a hepatoprotective effect, administered to a subject exhibiting said hepatitis, diabetes or metabolic syndrome, or to a subject at risk for hepatotoxicity, wherein said composition is selected from the group consisting of a pharmaceutical composition, a food composition or a beverage composition.

**15.** Composition for use in the treatment of hepatitis, diabetes or metabolic syndrome, comprising a vegetable extract

as defined in claim 13, particularly **characterized in that** this is administered to a subject exhibiting said hepatitis, diabetes or metabolic syndrome, or to a subject at risk for hepatotoxicity as a pharmaceutical composition comprising an extract of *Desmodium adscendens* that is enriched in D-pinitol; wherein said composition is selected from the group consisting of a pharmaceutical composition, a food composition and/or a beverage composition.

## Patentansprüche

1. Verfahren zum Herstellen eines Pflanzenextraktes, quantitativ bestimmt an D-Pinit, wobei eine Pflanze, *Desmodium adscendens,* aus der *Desmodium*-Familie ausgewählt wird, wobei eine Fraktion aus *Desmodium*-Pflanzenteilen extrahiert wird, wobei ein Pflanzenextrakt aus der Fraktion davon hergeleitet wird,
**dadurch gekennzeichnet, dass** ein gekennzeichneter Extrakt von *Desmodium adscendens* hergeleitet wird, von dem eine Zubereitung des Pflanzenextraktes quantitativ an D-Pinit bestimmt wird;
wobei der Pflanzenextrakt zwischen 0,1 und 10 %, bevorzugt 4 bis 5 %, D-Pinit enthält;
speziell wobei ein validiertes Analyseverfahren zur quantitativen Bestimmung von *Desmodium adscendens* an D-Pinit durchgeführt wird, wobei eine spezifische, quantitativ bestimmte *Desmodium-adscendens*-Zubereitung als *"Totum"* hergestellt wird, spezieller wobei eine biologisch kontrollierte Isolierung in dem Analyseverfahren durchgeführt wird, das unter Erzeugen einer Zusammensetzung durchgeführt wird, die für D-Pinit quantitativ bestimmt wird, wobei das Zusammensetzungsprodukt wiederholt angereichert wird und zahlreiche Fraktionen isoliert werden und wobei ein Dekokt gewonnen wird, das eine signifikante Menge D-Pinit enthält;
wobei der Extrakt getrennt wird und das isolierte Produkt als das methylierte Cyclit 3-O-Methyl-chiroinosit, d.h. D-Pinit, identifiziert wird,
dass ausgewählte Teile der Pflanze zuerst getrocknet werden und/oder dass die Pflanzenteile dann gemahlen oder fragmentiert, speziell zu Pulverform pulverisiert werden, spezieller wobei das Pulver als solches in eine Darreichungsform umgewandelt wird; wobei ausgehend von 1 kg getrocknete Pflanzenteile 60 bis 70 g, speziell etwa 65 g, Extrakt bzw. gemäß einem Verhältnis von 10 bis 20 : 1, spezieller 14 bis 16 : 1, gewonnen werden;
dass ein wässriges Dekokt der Pflanzenteile von *Desmodium adscendens* dann warm zu einem Dekokt hergestellt wird, indem eine bestimmte Menge getrockneter, wahlweise pulverisierter, Blätter in einer bestimmten Menge Wasser für eine bestimmte Zeitdauer gekocht wird und dass das wässrige Dekokt der Pflanzenteile von *Desmodium adscendens* dann abgekühlt wird, wonach die gewonnenen Portionen zusammengegeben und filtriert werden, wonach das gewonnene Filtrat eingeengt wird, speziell unter Vakuum, und dann lyophilisiert wird, um ein Lyophilisat zu bilden.

2. Verfahren nach dem vorhergehenden Anspruch, **dadurch gekennzeichnet, dass** die Bestandteile der Zusammensetzung unter Erfassung der Reproduzierbarkeit des Herstellungsverfahrens standardisiert werden,
und/oder dass die Hauptkomponenten der Bestandteile in *Desmodium adscendens* und die Gesamtmenge von D-Pinit mittels eines Analyseverfahrens dafür bestimmt werden, wobei das Extraktionsverfahren, die Produkte, die nach Extraktion gewonnen werden, und die Bedingungen bewertet und optimiert werden.

3. Verfahren nach einem der vorhergehenden Ansprüche zum Herstellen eines Pflanzenextraktes, der an D-Pinit angereichert ist, **dadurch gekennzeichnet, dass** es die Schritte umfasst:

   i) Extrahieren von Teilen einer Pflanze *Desmodium adscendens* mit Wasser, mit einem Niederalkylalkohol, vor allem einschließlich Methanol, Ethanol und/oder Isopropanol oder Kombinationen davon, oder mit einem Gemisch von einem oder mehreren Niederalkylalkoholen oder mit einem Gemisch von Wasser und einem oder mehreren Niederalkylalkoholen, mit einem Gemisch von Wasser, einem oder mehreren Niederalkylalkoholen und einem oder mehreren weniger polaren oder unpolaren organischen Lösemitteln, oder Kohlenstoffdioxid, um ein Dekokt zu gewinnen, das Pinit umfasst, und
   ii) Filtrieren und Einengen des Dekoktes; speziell wobei es ferner die Schritte umfasst:
   iii) Trennen des Dekoktes durch Säulenchromatographie, um mehrere Fraktionen zu gewinnen; und
   iv) Zusammengeben dieser Fraktionen.

4. Verfahren nach einem der vorhergehenden Ansprüche, **dadurch gekennzeichnet, dass** ein wässriges Dekokt der Pflanzenteile von *Desmodium adscendens* dann warm zu einem Dekokt hergestellt wird, indem eine bestimmte Menge getrockneter, wahlweise pulverisierter, ausgewählter Teile der Pflanze, die aus Blättern bestehen, in einer bestimmten Menge Wasser oder destilliertes Wasser für eine bestimmte Zeitdauer kochen lassen wird; speziell wobei das wässrige Dekokt der Pflanzenteile von *Desmodium adscendens* dann abgekühlt wird, wonach die gewonnenen Portionen zusammengegeben und filtriert werden, wonach das gewonnene Filtrat eingeengt wird und

dann lyophilisiert wird, um ein Lyophilisat zu bilden; oder wonach die resultierenden Portionen vereinigt und filtriert werden, wonach das Filtrat eingeengt und dann sprühgetrocknet wird; speziell wobei das Filtrat bzw. unter Vakuum eingeengt wird.

5. Verfahren nach einem der Ansprüche 1 bis 4, **dadurch gekennzeichnet, dass** ein wässriges Produkt als Mazerat der Pflanzenteile von *Desmodium adscendens* dann zu einem Extrakt kaltverarbeitet wird, speziell durch Einbinden einer bestimmten Menge getrockneter, wahlweise pulverisierter, Pflanzenteile, die in einer bestimmten Menge Wasser oder destilliertes Wasser für eine bestimmte Zeitdauer einzubeziehen sind.

6. Verfahren nach einem der Ansprüche 3 bis 5, **dadurch gekennzeichnet, dass** die Chromatographie über einer Sephadex LH-20 als Gelfiltrationsmedium mit einer Ausschlussgrenze von 4.000 bis 5.000 Dalton durchgeführt wird, wobei die Elution der Chromatographie mit Methanol durchgeführt wird.

7. Verfahren nach einem der Ansprüche 3 bis 6, **dadurch gekennzeichnet, dass** die Fraktionen gemäß ihrem chromatographischen Muster in eine Anzahl von Unterfraktionen, speziell 11, zusammengegeben werden, speziell wobei die zusammengegebenen Fraktionen diejenigen sind, die mit 1 % Anisaldehyd in Schwefelsäure reagieren und ein Produkt mit einer grünen Farbe liefern;
wobei die Unterfraktionen 5 bis 11, speziell 200 mg, die einen Fleck mit einer grünen Farbe aufweisen, zusammengegeben werden, nach dem Besprühen mit Anisaldehyd 1 % $H_2SO_4$ in MeOH und Erhitzen auf etwa 120 °C für 10 Min, wobei die Fraktion weiterer Säulenchromatographie unterworfen wird, mit MeOH eluiert wird, wobei bestimmte Fraktionen, speziell von 100 ml, noch einmal aufgefangen und analysiert werden,
wobei die Unterfraktionen 4 bis 5, speziell 120 mg, aus dieser Säule zusammengegeben werden, wobei die Letztgenannten einer weiteren Säulenchromatographie unter den gleichen Bedingungen unterworfen werden, wonach ein Reinprodukt gewonnen wird, speziell weiß;
spezieller ferner umfassend die Schritte:
v) Trennen der zusammengegebenen Fraktionen durch Gelfiltrationschromatographie; noch spezieller wobei die Chromatographie über einer Sephadex LH-20 als Gelfiltrationsmedium mit einer Ausschlussgrenze von 4.000 bis 5.000 Dalton durchgeführt wird und die Elution mit Methanol durchgeführt wird.

8. Verfahren nach einem der vorhergehenden Ansprüche, **gekennzeichnet durch** eine Ausweitung der Kennzeichnung der *Desmodium-adscendens*-Zubereitung mit einem Flavonoidprofil, speziell wobei Vitexin identifiziert wird, wobei sich die Gesamtmenge von Flavonoiden, definiert als die Summe aus den erhaltenen Signalen, ausgedrückt als Vitexin, auf etwa 1,05 % beläuft, in einem typischen Lyophilisat des Dekoktes von *Desmodium adscendens,* als Flavonoidanteil und -profil im Lyophilisat von *Desmodium adscendens* zwischen 0,1 und 5 %, spezieller wobei der Anteil in der Größenordnung von 1 % ist.

9. Verfahren nach einem der vorhergehenden Ansprüche, speziell 3 bis 8, spezieller 7, zum Aufreinigen von D-Pinit aus einer Pflanze, **dadurch gekennzeichnet, dass** es umfasst:

i) Extrahieren von Teilen einer Pflanze *Desmodium adscendens* mit Wasser, mit einem Niederalkylalkohol, mit einem Gemisch von einem oder mehreren Niederalkylalkoholen oder mit einem Gemisch von Wasser und einem oder mehreren Niederalkylalkoholen, mit einem Gemisch von Wasser, oder Kohlenstoffdioxid, um ein Dekokt zu gewinnen, das Pinit umfasst, und
ii) Filtrieren und Einengen des Dekoktes;
iii) Trennen des Dekoktes durch Chromatographie, um mehrere Fraktionen zu gewinnen;
iv) Zusammengeben derjenigen Fraktionen, die mit 1 % Anisaldehyd in Schwefelsäure reagieren und ein Produkt mit einer grünen Farbe liefern; wobei
die Unterfraktionen 5 bis 11, speziell 200 mg, die einen Fleck mit einer grünen Farbe aufweisen, zusammengegeben werden, nach Besprühen mit Anisaldehyd 1 % $H_2SO_4$ in MeOH und Erhitzen auf etwa 120 °C für 10 Min, wobei die Fraktion weiterer Säulenchromatographie unterworfen wird, mit MeOH eluiert wird, wobei bestimmte Fraktionen, speziell von 100 ml, noch einmal aufgefangen und analysiert werden;
v) Trennen der pinithaltigen Fraktion durch Gelfiltrationschromatographie; speziell weiß, speziell wobei die Chromatographieschritte unter Benutzen einer Sephadex LH-20 als Medium mit einer Ausschlussgrenze von 4.000 bis 5.000 Dalton durchgeführt wird und die Elution mit Methanol durchgeführt wird; spezieller wobei das Produkt D-Pinit ist;
wobei die Unterfraktionen 4 bis 5, speziell 120 mg, aus dieser Säule zusammengegeben werden, wobei die Letztgenannten einer weiteren Säulenchromatographie unter den gleichen Bedingungen unterworfen werden, wonach ein Reinprodukt gewonnen wird, speziell weiß; und

wobei der Extrakt getrennt wird und das isolierte Produkt als das methylierte Cyclit 3-O-Methyl-chiroinosit, d.h. D-Pinit, identifiziert wird.

**10.** Verfahren nach einem der Ansprüche 3 bis 9, **dadurch gekennzeichnet, dass** Zuckeralkohole, speziell Xylit, vor allem in der Gaschromatographie, oder möglicherweise andere, wie z.B. Sorbit, Mannit, wahlweise Dulcit und Inosit, anstelle des oben erwähnten Xylits als ein innerer Standard ausgewählt werden, mit den zwei Letztgenannten in einem geringeren Maße.

**11.** Verfahren nach einem der Ansprüche 1 bis 10, **dadurch gekennzeichnet, dass** ein standardisierter Extrakt aus der Pflanze *Desmodium adscendens* hergeleitet wird, für D-Pinit in seiner Wirkung gegen Hepatitis, speziell in vorbeugender oder möglicherweise heilender Wirkung, mit der Isolierung von D-Pinit aus *Desmodium adscendens* quantitativ bestimmt wird, wobei D-Pinit einen Wirkstoff bildet, speziell hinsichtlich Leberschaden chemischen, physikalischen, infektiösen oder immunologischen Ursprungs.

**12.** Pflanzenextrakt, gewonnen durch ein Verfahren, wie in einem der vorhergehenden Ansprüche definiert, zum Schützen der Leber in einem Säuger, zur Vorbeugung einer Lebererkrankung in einem Säuger und/oder zur Behandlung davon;
speziell wobei er aus der Pflanze *Desmodium adscendens* hergeleitet ist, an dem Molekül D-Pinit, das gegen Hepatitis wirkt, quantitativ bestimmt ist, wobei D-Pinit als Wirkstoff aus *Desmodium adscendens* isoliert ist, standardisierte Formen; spezieller wobei der Pflanzenextrakt zwischen 0,1 und 10 %, bevorzugt 4 bis 5 %, D-Pinit enthält.

**13.** Pharmazeutische Zusammensetzung, umfassend den Extrakt nach Anspruch 12.

**14.** Zusammensetzung zur Benutzung als ein Medikament, umfassend einen Pflanzenextrakt, wie in Anspruch 13 definiert, speziell **dadurch gekennzeichnet, dass** dieses als ein Antioxidationsmittel für spezifische Zielgruppen eingesetzt wird, speziell wobei das bezeichnete Ziel von einem Menschen, spezieller Alkoholikern und/oder Patienten mit einem metabolischen Syndrom, noch spezieller Diabetespatienten mit Typ-2-Diabetes gebildet wird, speziell zum Behandeln von Hepatitis, Diabetes oder metabolischem Syndrom oder zum Erzielen eines leberschützenden Effektes, verabreicht einem Probanden, der die Hepatitis, den Diabetes oder das metabolische Syndrom aufweist, oder einem Probanden mit Risiko für Lebertoxizität, wobei die Zusammensetzung aus der Gruppe ausgewählt ist, die aus einer pharmazeutischen Zusammensetzung, einer Nahrungsmittelzusammensetzung oder einer Getränkezusammensetzung besteht.

**15.** Zusammensetzung zur Benutzung in der Behandlung von Hepatitis, Diabetes oder metabolischem Syndrom, umfassend einen Pflanzenextrakt, wie in Anspruch 13 definiert, speziell **dadurch gekennzeichnet, dass** diese einem Probanden, der die Hepatitis, den Diabetes oder das metabolische Syndrom aufweist, oder einem Probanden mit Risiko für Lebertoxizität als eine pharmazeutische Zusammensetzung verabreicht wird, umfassend einen Extrakt von *Desmodium adscendens,* der an D-Pinit angereichert ist; wobei die Zusammensetzung aus der Gruppe ausgewählt ist, die aus einer pharmazeutischen Zusammensetzung, einer Nahrungsmittelzusammensetzung und/oder einer Getränkezusammensetzung besteht.

## Revendications

**1.** Procédé de production d'un extrait de plante, quantifié sur du D-pinitol, dans lequel une plante *Desmodium adscendens* est sélectionnée parmi la famille de *Desmodium,* dans laquelle une fraction est extraite de parties de plante *Desmodium,* dans laquelle un extrait de plante est dérivé de ladite fraction de celle-ci, **caractérisé en ce qu'**un extrait caractérisé est dérivé de *Desmodium adscendens,* à partir duquel une préparation dudit extrait de plante est quantifiée sur le D-pinitol; dans lequel ledit extrait de plante contient entre 0,1 et 10%, de préférence de 4 à 5% de D-pinitol;
en particulier dans lequel une méthode analytique validée est mise en oeuvre pour ladite quantification de *Desmodium adscendens* sur le D-pinitol, dans lequel une préparation spécifique de *Desmodium adscendens* quantifié est produite sous forme de « totum », plus particulièrement dans lequel un isolement contrôlé biologiquement est effectué dans ladite méthode analytique qui est réalisée en engendrant une composition quantifiée pour le D-pinitol, dans laquelle le produit de composition est enrichi à plusieurs reprises et de nombreuses fractions sont isolées, et dans lequel une décoction est obtenue qui contient une quantité significative de D-pinitol; dans lequel ledit extrait est séparé et le produit isolé est identifié en tant que 3-O-méthyl-chiro-inositol de cyclitol méthylé, c'est-à-dire du D-pinitol,
**en ce que** des parties sélectionnées de ladite plante sont d'abord séchées, et/ou **en ce que** lesdites parties de

plante sont ensuite broyées ou fragmentées, en particulier pulvérisées sous forme de poudre, plus particulièrement dans lequel ladite poudre est convertie en tant que telle sous une forme pharmaceutique; dans laquelle à partir de 1 kg de parties végétales séchées, on obtient de 60 à 70 g, en particulier environ 65 g d'extrait, respectivement selon un rapport de 10-20:1, plus particulièrement 14-16:1;
**en ce qu'**une décoction aqueuse desdites parties végétales de *Desmodium adscendens* est ensuite préparée à chaud en une décoction en faisant bouillir une certaine quantité de feuilles séchées, éventuellement en poudre, suivant une certaine quantité d'eau pendant une certaine durée et **en ce que** ladite décoction aqueuse desdites parties végétales de *Desmodium adscendens* est ensuite refroidie, après quoi les portions obtenues sont combinées et filtrées, après quoi le filtrat obtenu est concentré, notamment sous vide, puis lyophilisé pour former un lyophilisat.

**2.** Procédé selon la revendication précédente, **caractérisé en ce que** les constituants de ladite composition sont standardisés avec capture de la reproductibilité du procédé de fabrication, et/ou **en ce que** les principaux composants des constituants du *Desmodium adscendens* et la quantité totale de D-pinitol sont déterminés au moyen d'une méthode analytique à cet effet, dans laquelle le processus d'extraction, les produits obtenus après extraction et les conditions sont évalués et optimisés.

**3.** Procédé selon l'une quelconque des revendications précédentes pour produire un extrait végétal enrichi du D-pinitol, **caractérisé en ce qu'**il comprend les étapes consistant à

i) extraire des parties d'une plante de *Desmodium adscendens* avec de l'eau, avec un alcool alkylique inférieur, comprenant notamment du méthanol, de l'éthanol et/ou de l'isopropanol, ou des combinaisons de ceux-ci, ou avec un mélange d'un ou plusieurs alcools alkyliques inférieurs, ou avec un mélange d'eau et un ou plusieurs alcools alkyliques inférieurs, avec un mélange d'eau, d'un ou plusieurs alcools alkyliques inférieurs et un ou plusieurs solvants organiques moins polaires ou non polaires, ou du dioxyde de carbone, pour obtenir une décoction comprenant du pinitol, et
ii) filtrer et concentrer ladite décoction;
en particulier dans lequel il comprend en outre les étapes de
iii) séparer la décoction par chromatographie sur colonne pour obtenir plusieurs fractions; et
iv) combiner ces fractions.

**4.** Procédé selon l'une quelconque des revendications précédentes, **caractérisé en ce qu'**une décoction aqueuse desdites parties de plantes de *Desmodium adscendens* est ensuite préparée à chaud en une décoction en faisant bouillir une certaine quantité de parties sélectionnées séchées, éventuellement en poudre, de ladite plante constituée de feuilles dans une certaine quantité d'eau ou d'eau distillée, pendant une certaine durée; en particulier dans lequel ladite décoction aqueuse desdites parties de plantes de *Desmodium adscendens* est ensuite refroidie, après quoi les portions obtenues sont combinées et filtrées, après quoi le filtrat obtenu est concentré, puis lyophilisé pour former un lyophilisat; ou après quoi les parties résultantes sont rassemblées et filtrées, après quoi le filtrat est concentré, puis séché par pulvérisation; en particulier dans lequel le filtrat est concentré sous vide respectivement.

**5.** Procédé selon l'une quelconque des revendications 1 à 4, **caractérisé en ce qu'**un produit aqueux en tant que macérat desdites parties de plantes de *Desmodium adscendens* est ensuite transformé à froid en un extrait, notamment en incorporant une certaine quantité de parties de plantes séchées, éventuellement pulvérisée à inclure dans une certaine quantité d'eau ou d'eau distillée, pendant une certaine durée.

**6.** Procédé selon l'une quelconque des revendications 3 à 5, **caractérisé en ce que** la chromatographie est effectuée par un Sephadex LH-20 comme milieu de filtration de gel ayant une limite d'exclusion de 4000-5000 daltons, dans lequel l'élution de la chromatographie est réalisée avec du méthanol.

**7.** Procédé selon l'une des revendications 3 à 6, **caractérisé en ce que** lesdites fractions sont combinées en un certain nombre de sous-fractions, notamment 11, selon leur motif chromatographique, notamment dans lequel les fractions combinées sont celles qui réagissent avec 1% d'anisaldéhyde dans l'acide sulfurique pour donner un produit ayant une couleur verte;
dans lequel lesdites sous-fractions 5-11, en particulier 200 mg, qui ont une tache de couleur verte, sont combinées, après pulvérisation d'anisaldéhyde à 1% de $H_2SO_4$, dans du MeOH et chauffage à environ 120°C pendant 10 min, dans lequel ladite fraction est soumise à une nouvelle chromatographie sur colonne éluée au MeOH, dans lequel certaines fractions, en particulier de 100 ml, sont à nouveau collectées et analysées,
dans lequel lesdites sous-fractions 4-5, en particulier 120 mg, de cette colonne sont combinées, dans lequel ces dernières sont soumises à une nouvelle chromatographie sur colonne dans les mêmes conditions, après quoi un

produit pur est obtenu, en particulier blanc;
comprenant plus particulièrement les étapes de
v) séparation des fractions combinées par chromatographie de filtration sur gel; encore plus particulièrement dans lequel la chromatographie est effectuée sur un Sephadex LH-20 comme milieu de filtration sur gel ayant une limite d'exclusion de 4000-5000 daltons et l'élution est effectuée avec du méthanol.

**8.** Procédé selon l'une quelconque des revendications précédentes, **caractérisé par** une extension de la caractérisation de la préparation de *Desmodium adscendens* ayant un profil flavonoïde, en particulier dans lequel de la vitexine est identifiée dans laquelle la quantité totale de flavonoïdes, définie comme la somme des signaux obtenus, exprimée comme vitexine, s'élève à environ 1,05%, dans un lyophilisat typique de la décoction de *Desmodium adscendens,* comme taux de flavonoïdes et profil dans le lyophilisat de *Desmodium adscendens* entre 0,1 et 5%, plus particulièrement dans lequel ledit taux est de l'ordre de 1%.

**9.** Procédé selon l'une quelconque des revendications précédentes, en particulier 3 à 8, plus particulièrement 7, pour purifier le D-pinitol à partir d'une plante, **caractérisé en ce qu'**il comprend:

i) l'extraction de parties d'une plante de *Desmodium adscendens* avec de l'eau, avec un alcool alkylique inférieur, avec un mélange d'un ou plusieurs alcools alkyliques inférieurs, ou avec un mélange d'eau et d'un ou plusieurs alcools alkyliques inférieurs, avec un mélange d'eau, ou du dioxyde de carbone, pour obtenir une décoction comprenant du pinitol, et
ii) filtrer et concentrer ladite décoction;
iii) séparer la décoction par chromatographie pour obtenir plusieurs fractions;
iv) combiner ces fractions qui réagissent avec 1% d'anisaldéhyde dans l'acide sulfurique pour donner un produit ayant une couleur verte; dans lequel les sous-fractions 5-11, en particulier 200 mg, qui ont une tache de couleur verte, sont réunies, après pulvérisation d'anisaldéhyde à 1% de $H_2SO_4$ dans du MeOH et après chauffage à environ 120°C pendant 10 min, ladite fraction étant soumise à une autre chromatographie sur colonne éluée avec du MeOH, certaines fractions, en particulier de 100 ml, étant à nouveau collectées et analysées;
v) séparation de la fraction contenant du pinitol par chromatographie de filtration sur gel; en particulier blanc, en particulier dans lequel les étapes de chromatographie sont effectuées avec utilisation d'un Sephadex LH-20 comme milieu ayant une limite d'exclusion de 4000-5000 daltons et l'élution est effectuée avec du méthanol; plus particulièrement dans lequel le produit est le D-pinitol;
dans lequel les sous-fractions 4-5, en particulier 120 mg, de cette colonne sont combinées, dans lequel ces dernières sont soumises à une nouvelle chromatographie sur colonne dans les mêmes conditions, après quoi un produit pur est obtenu, en particulier blanc; et
dans lequel ledit extrait est séparé et le produit isolé est identifié en tant que 3-O-méthyl-chiro-inositol de cyclitol méthylé, c'est-à-dire le D-pinitol.

**10.** Procédé selon l'une des revendications 3 à 9, **caractérisé en ce que** des alcools de sucre sont choisis comme étalon interne, en particulier du xylitol, notamment en chromatographie en phase gazeuse, ou éventuellement d'autres tels que le sorbitol, le mannitol, éventuellement le dulcitol et l'inositol, au lieu du xylitol susmentionné, les deux derniers dans une moindre mesure.

**11.** Procédé selon l'une quelconque des revendications 1 à 10, **caractérisé en ce qu'**un extrait standardisé est dérivé de la plante *Desmodium adscendens,* quantifiée pour le D-pinitol dans son action contre l'hépatite, notamment en action préventive ou éventuellement curative, avec l'isolement du D-pinitol à partir du *Desmodium adscendens,* dans lequel le D-pinitol forme un ingrédient actif, en particulier en ce qui concerne les dommages au foie d'origine chimique, physique, infectieuse ou immunologique.

**12.** Extrait de plante obtenu par un procédé tel que défini dans l'une des revendications précédentes pour protéger le foie chez un mammifère, pour la prévention d'une maladie du foie chez un mammifère et/ou pour son traitement; en particulier dans lequel il est dérivé du *Desmodium adscendens* quantifié sur la molécule de D-pinitol active contre l'hépatite, dans lequel le D-pinitol est isolé du *Desmodium adscendens* sur un principe actif, de formes standardisées; plus particulièrement dans lequel ledit extrait de plante contient entre 0,1 et 10%, de préférence de 4 à 5% de D-pinitol.

**13.** Composition pharmaceutique comprenant l'extrait de la revendication 12.

**14.** Composition à utiliser comme médicament comprenant un extrait végétal tel que défini à la revendication 13, **caractérisée** en particulier en ce qu'elle est utilisée comme agent anti-oxydant pour des groupes cibles spécifiques,

particulièrement lorsque la cible visée est formée par l'homme, plus particulièrement les alcooliques et/ou par des patients atteints d'un syndrome métabolique, encore plus particulièrement par des patients diabétiques de type 2, en particulier pour traiter l'hépatite, le diabète ou le syndrome métabolique, ou pour obtenir un effet hépatoprotecteur, administrée à un sujet présentant lesdites hépatite, diabète ou syndrome métabolique, ou à un sujet à risque d'hépatotoxicité, ladite composition étant choisie dans le groupe consistant en une composition pharmaceutique, une composition alimentaire ou une composition de boisson.

**15.** Composition à utiliser dans le traitement de l'hépatite, du diabète ou du syndrome métabolique, comprenant un extrait végétal tel que défini à la revendication 13, particulièrement **caractérisé en ce qu'**il est administré à un sujet présentant lesdites hépatite, diabète ou syndrome métabolique ou à un sujet à risque d'hépatotoxicité en tant que composition pharmaceutique comprenant un extrait de *Desmodium adscendens* enrichi au D-pinitol; ladite composition étant choisie dans le groupe consistant en une composition pharmaceutique, une composition alimentaire et/ou une composition de boisson.

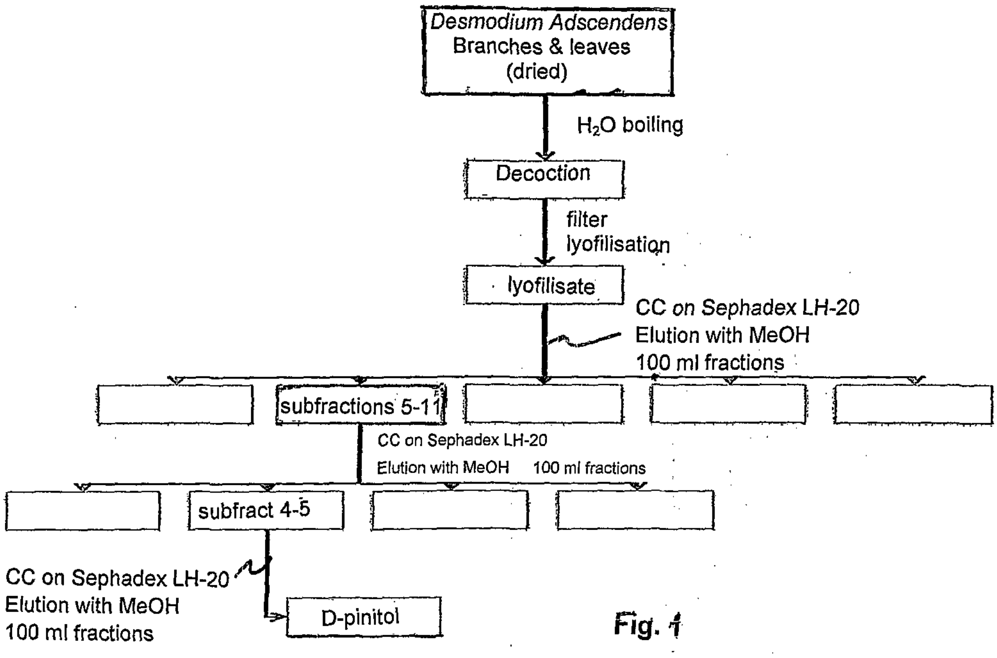


Fig. 1

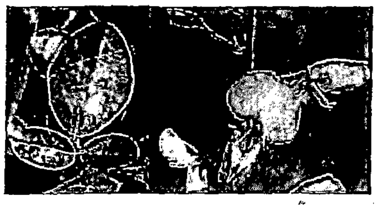

Fig. 2

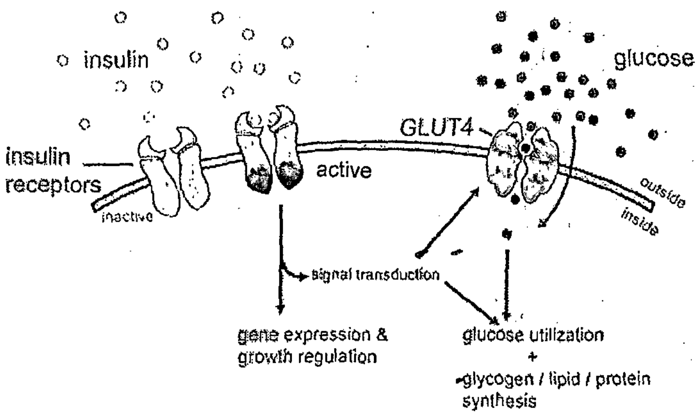


Fig. 3

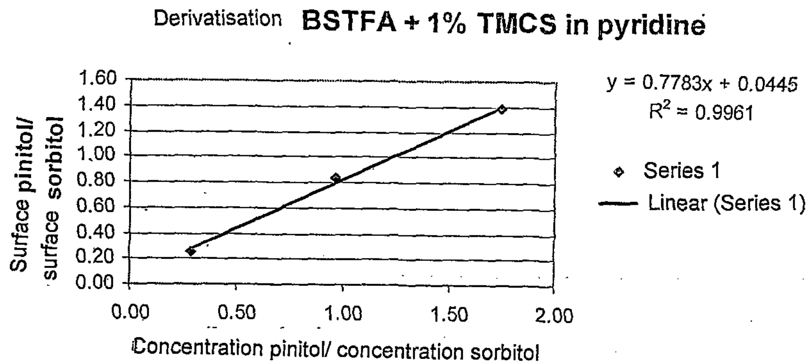


Fig. 4

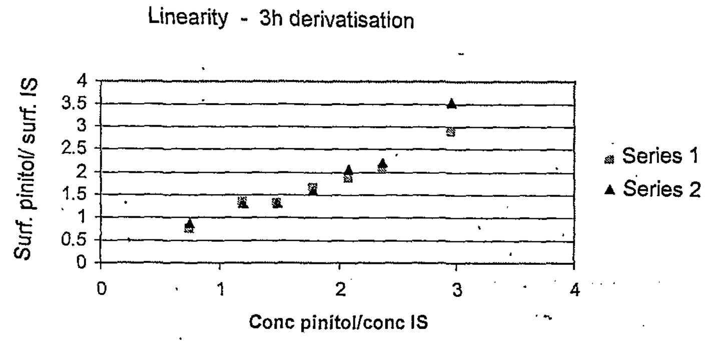


Fig. 5

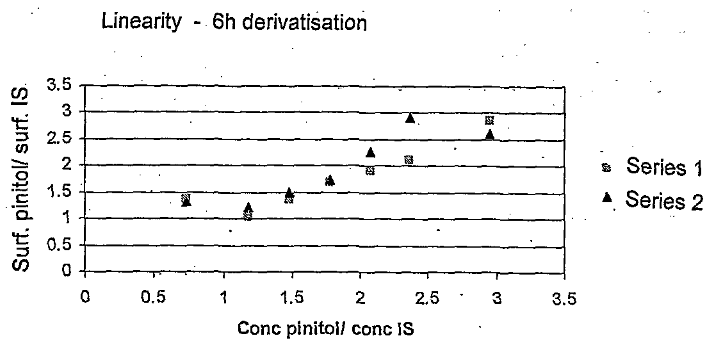


Fig. 6

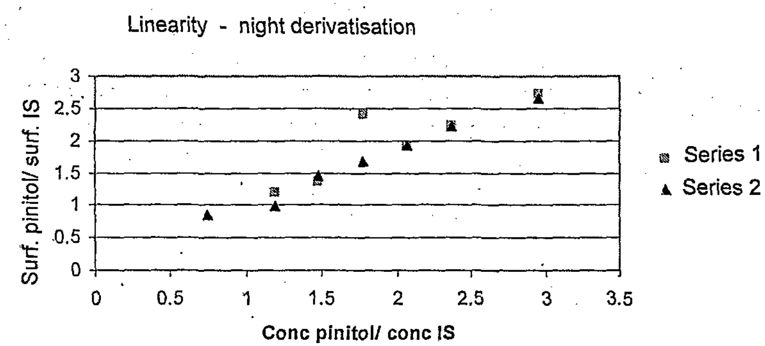


Fig. 7

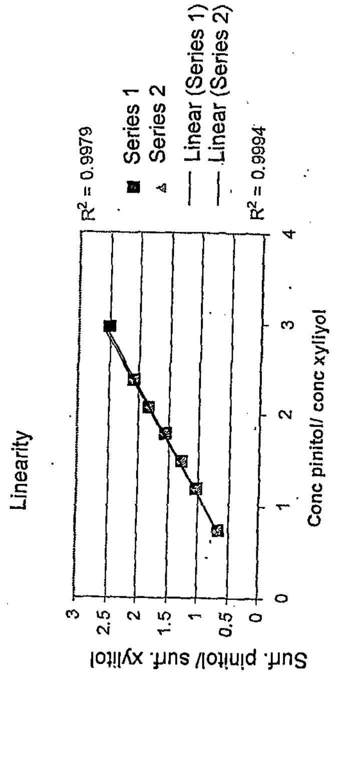


Fig. 8

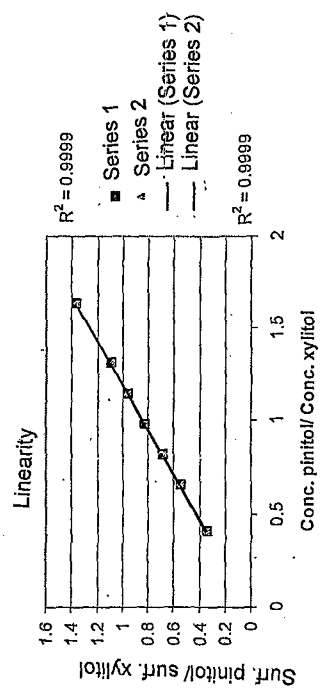


Fig. 9

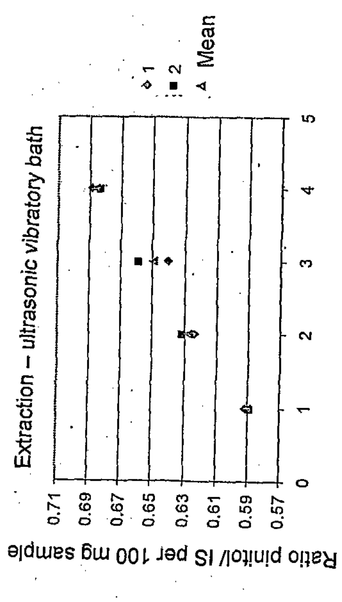


Fig. 10

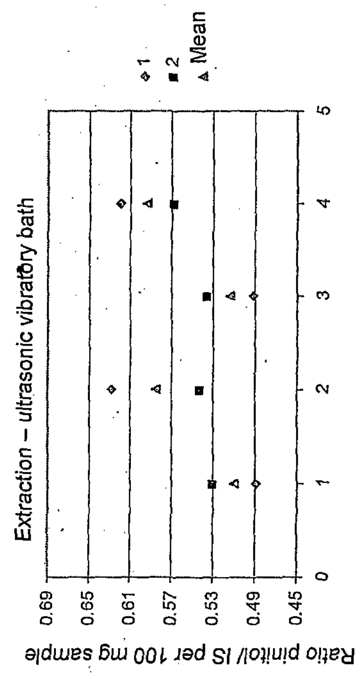


Fig. 11

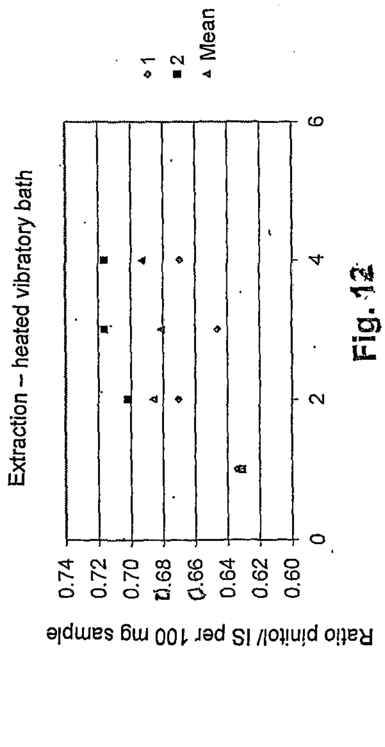


Fig. 12

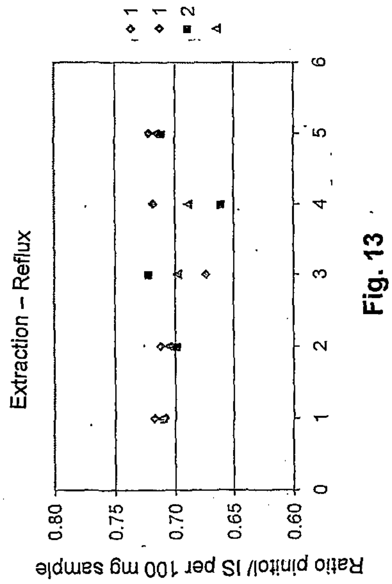


Fig. 13

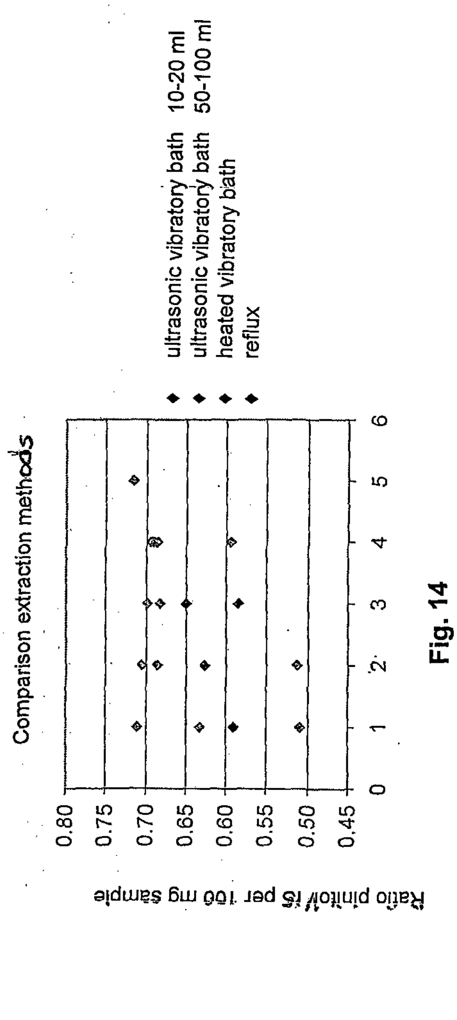


Fig. 14

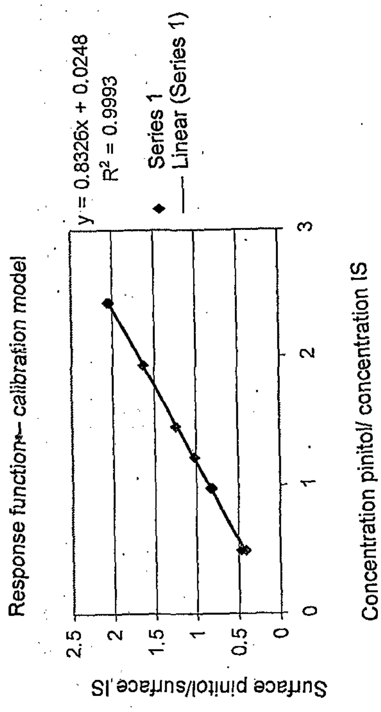


Fig. 15

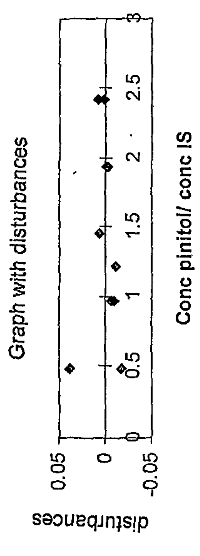


Fig. 16

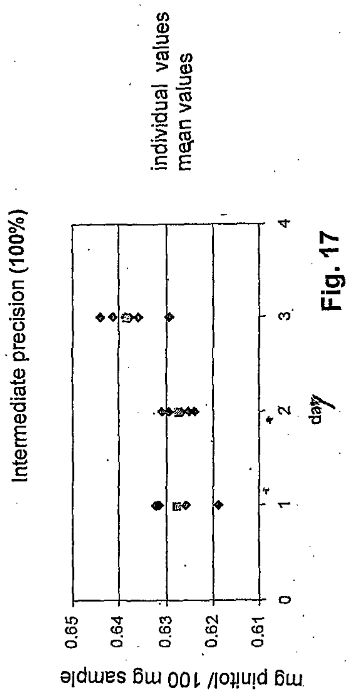


day

Fig. 17

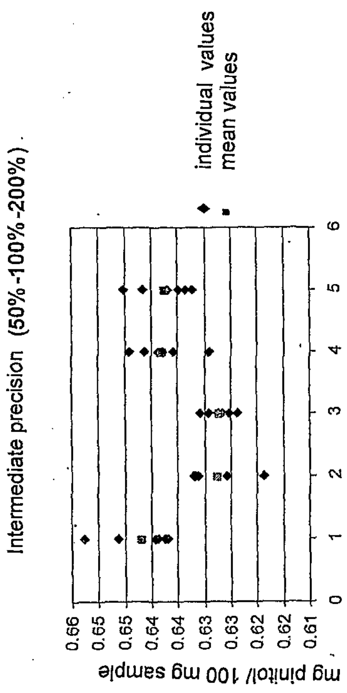


Fig. 18

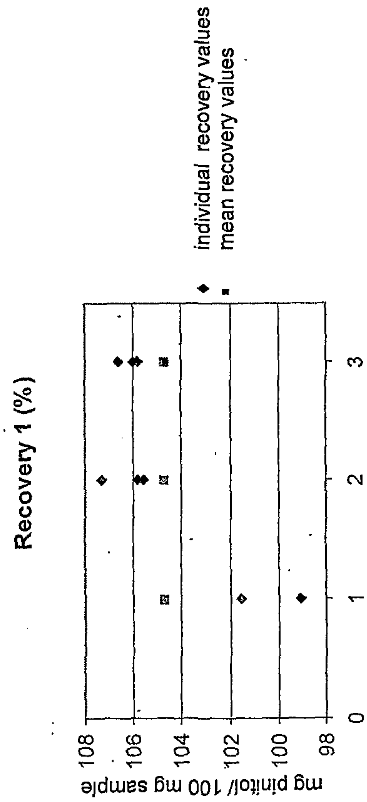


Fig. 19

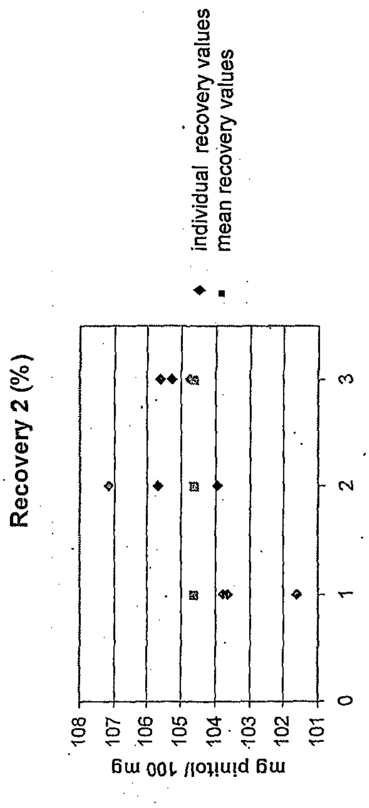


Fig. 20

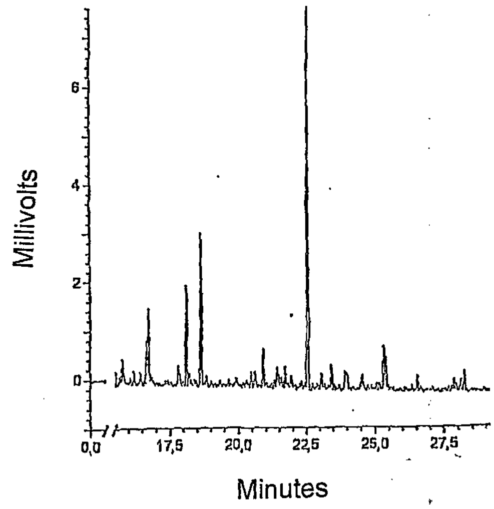


Fig. 21

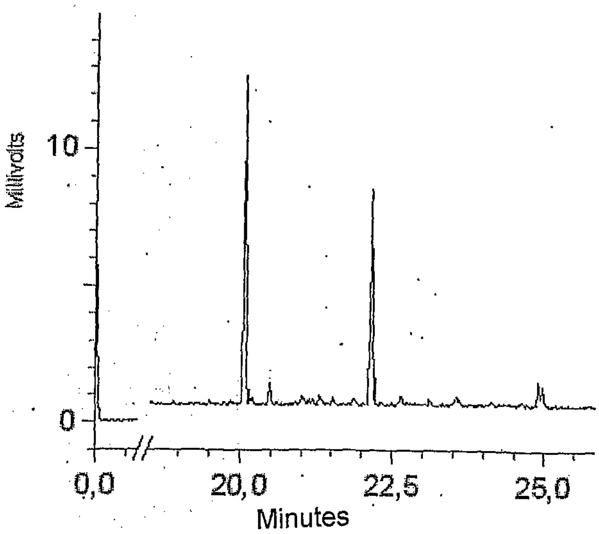


Fig. 22

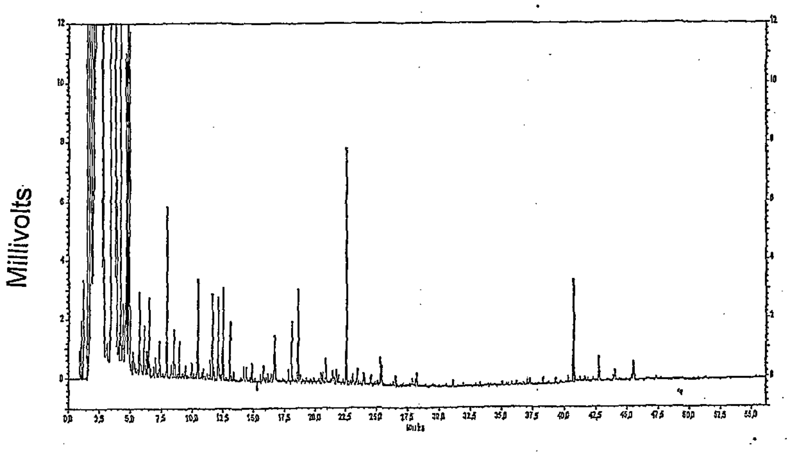


Fig. 23

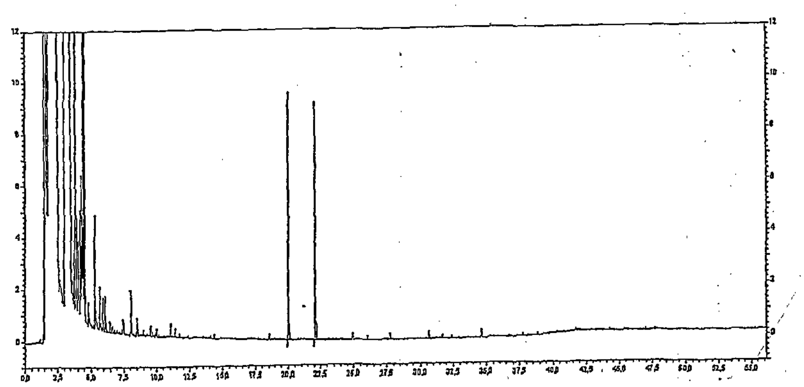

Fig. 24

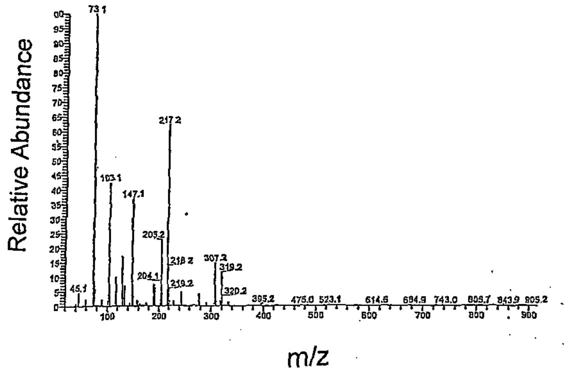


Fig. 25

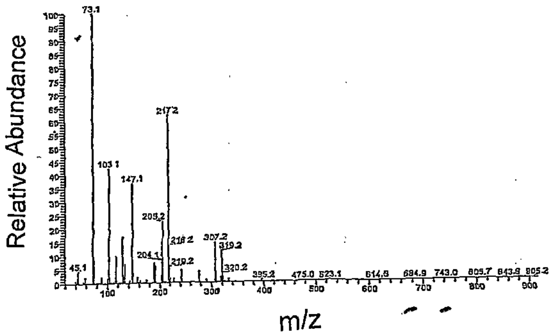


Fig. 26

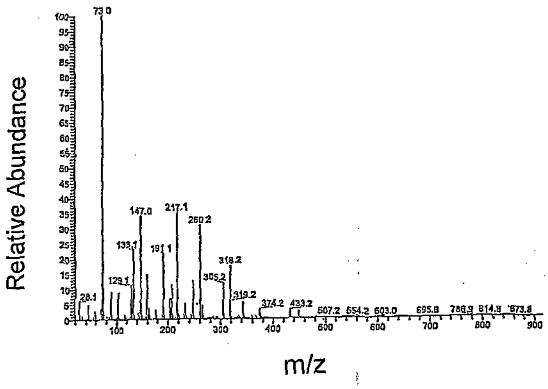


Fig. 27

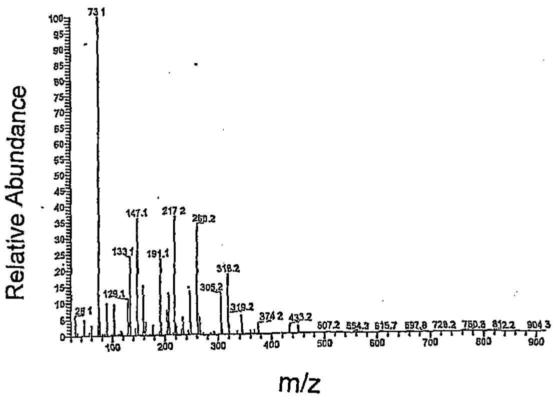


Fig. 28

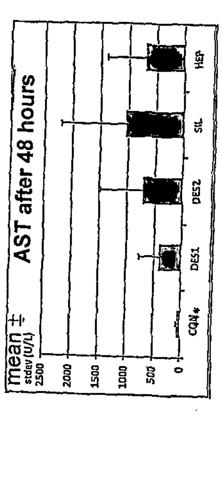


Fig. 29

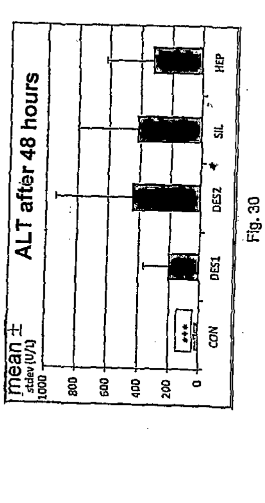


Fig. 30

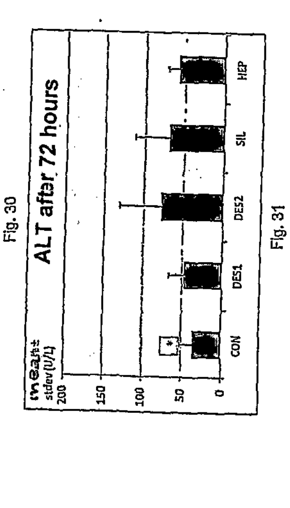


Fig. 31

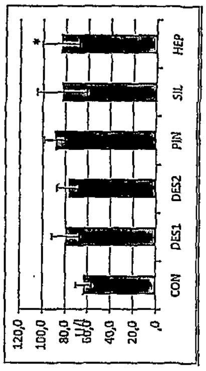


Fig. 32

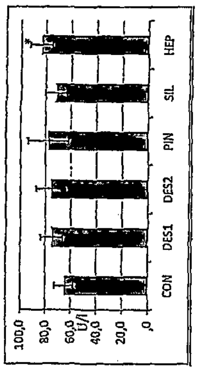


Fig. 33

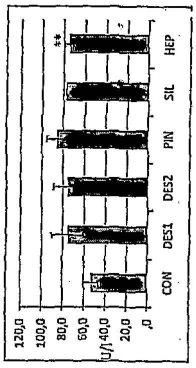


Fig. 34

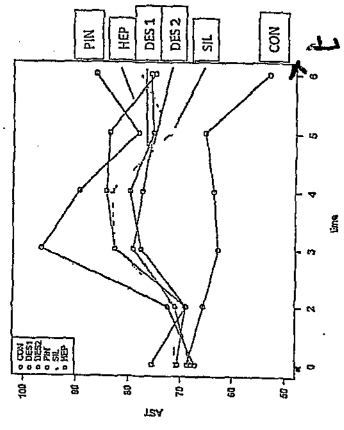


Fig. 35

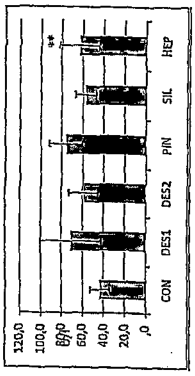


Fig. 36

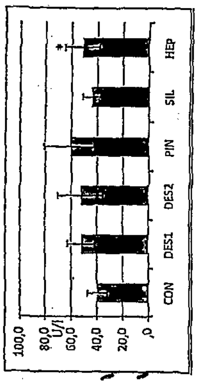


Fig. 37

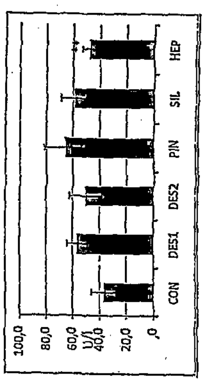


Fig. 38

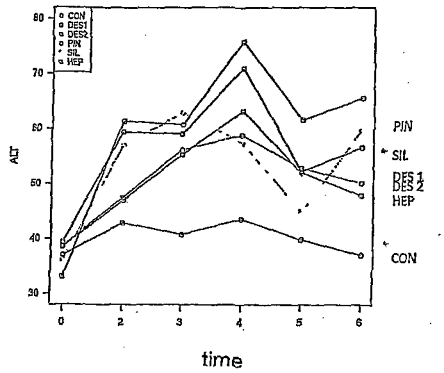


Fig. 39

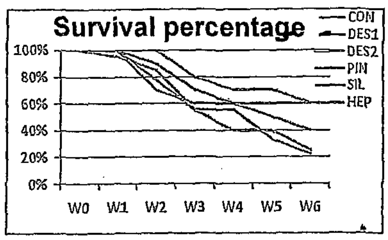


Fig. 40

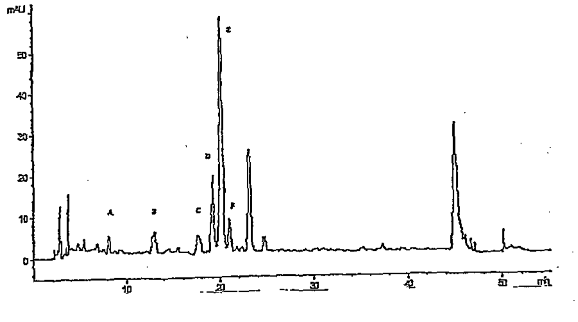

Fig. 41 : Chromatographic profile

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0309342 A1
- WO 2004084875 A1
- WO 2004084875 A


### Non-patent literature cited in the description


- **MUANDA et al.** *Evidence-Based Complementary and Alternative Medicine,* 2011, vol. 2011, 620862-1, 620862-9
- **BEVERIDGE et al.** *Aust. J. Chem.,* 1977, vol. 30, 1583-1590
- **FORD et al.** *Aust. J. Agric. Res.,* 1978, vol. 29, 963-974
- *Zhongcaoyao,* 2007, vol. 38 (8), 1157-1159
- *Curr. Sci.,* 1982, vol. 51, 936-937
- *Current Science,* 1987, vol. 56 (3), 139-141